# EUROPÄISCHE PATENTSCHRIFT

(11) **EP 3 481 334 B1**
(45) Veröffentlichungstag und Bekanntmachung des Hinweises auf die Patenterteilung: **15.06.2022**
(21) Anmeldenummer: 17809174.0
(22) Anmeldetag: 05.07.2017
(51) Int. Cl.: A61F 2/16

(54) **MAGAZIN FÜR EINEN INJEKTOR ZUM IMPLANTIEREN EINER INTRAOKULARLINSE**
CARTRIDGE FOR AN INJECTOR FOR IMPLANTING AN INTRAOCULAR LENS
CASSETTE D'UN INJECTEUR DESTINÉ À L'IMPLANTATION D'UNE LENTILLE INTRAOCULAIRE

(30) Priorität: 07.07.2016 DE 102016008195
(43) Veröffentlichungstag der Anmeldung: 15.05.2019
(73) Patentinhaber: Iolution GmbH, 22761 Hamburg (DE)
(72) Erfinder: MAROSCHEK, Christoph, 22761 Hamburg (DE)
(74) Vertreter: Friderichs, Gunther
(86) Internationale Anmeldenummer: PCT/DE2017/000193
(87) Internationale Veröffentlichungsnummer: WO 2018/006889

(56) Entgegenhaltungen:
- EP-A1- 2 926 770
- WO-A1-2012/155887
- WO-A2-2018/006889
- US-A1- 2008 058 830
- US-A1- 2014 066 946

## Beschreibung

### Beschreibung der Erfindung

Die vorliegende Erfindung betrifft ein Magazin für ein Injektorsystem zum Implantieren einer Intraokularlinse in ein Auge.

### Hintergrund der Erfindung

Intraokularlinsen stellen Linsenimplantate oder künstliche Linsen zum Ersatz der natürlichen Linsen eines menschlichen Auges dar. Sie werden insbesondere als Ersatz für die unter einer Augentrübung (Katarakt) erkrankten Linsen des Auges eingesetzt. Mittels einer Operation werden die erkrankten Linsen entfernt und die Intraokularlinsen eingesetzt. Diese werden zum Beispiel mittels eines sogenannten Injektors in das Auge eingesetzt bzw. eingeführt. Hierbei ist es von Bedeutung, dass der Operationsschnitt, durch welchen eine Intraokularlinse implantiert wird, möglichst klein ist (zum Beispiel etwa 2,2 mm). Dadurch kann ein möglichst schneller und komplikationsfreier Heilungsprozess gewährleistet werden. Gegebenenfalls ist auch keine Naht erforderlich.

Um Intraokularlinsen, die im Allgemeinen einen Durchmesser von ca. 5 bis 6 mm besitzen, implantieren zu können, müssen diese faltbar sein, damit sie durch den kleinen Schnitt von etwa 2,2 mm hindurchpassen.

Ein Injektor zum Falten und Einbringen einer gefalteten Linse in das menschliche Auge ist zum Beispiel in der internationalen Patentanmeldung WO 00/45746 A1 beschrieben. Der Inhalt dieser Patentanmeldung wird in die vorliegende Patentanmeldung durch Bezugnahme vollständig inkorporiert. Dort ist ein Injektor zum Implantieren bzw. Einbringen einer zeitweilig gefalteten Intraokularlinse beschrieben, mit welchem die gefaltete Linse durch eine Schnittöffnung im Auge mit der erforderlichen Größe von etwa 2,2 mm in die Linsenkapsel des Auges einsetzbar ist. In einer sogenannten Einbringöffnung liegt die ungefaltete Linse für den Transport flach auf einer Auflagefläche auf und wird über eine längsmittig zur Linse sich erstreckende Halterippe festgehalten. Die Halterippe wird hier auch zum Falten und Einbringen der Linse in den Transportkanal verwendet.

Der dort beschriebene Injektor gewährleistet ein sicheres Implantieren einer Intraokularlinse in ein menschliches und/oder tierisches Auge. Das Laden des Injektors bzw. das Einlegen der Intraokularlinse in den Injektor kann aber erst kurz vor der Operation durch den operierenden Augenarzt oder eine unterstützende OP-Schwester erfolgen.

Um die Intraokularlinse präzise in das Auge einbringen zu können, ist es aber erforderlich, dass die Linse möglichst präzise auf der Auflagefläche positioniert ist, so dass die Linse durch die Faltrippe präzise in den Transportkanal einbringbar ist.

Wird die Linse nicht ausreichend präzise geladen, kann dies zu einer unerwünschten Rotation der Linse beim Falten und Einbringen der Linse in den Transportkanal mittels der Faltrippe führen. Unter Umständen kann hierbei sogar die Linse derart geklemmt zwischen der Faltrippe und dem Transportkanal werden, dass diese unbrauchbar wird.

Daher stellt das Laden des Injektors durch den Augenarzt oder die OP-Schwester ein potentielles Risiko dar. Zudem kann die bis dahin in einer Verpackung steril gelagerte Linse beim Entnehmen aus der Verpackung und beim Einlegen in den Injektor verunreinigt werden.

Eine Weiterentwicklung des vorab beschriebenen Injektors ist in der internationalen Patentanmeldung WO 2012/155887 A1 gezeigt. Der Inhalt dieser Patentanmeldung wird in die vorliegende Patentanmeldung durch Bezugnahme vollständig inkorporiert. Die Linse kann hier bereits vorab in dem Injektor gelagert werden. Die Halterung der Linse und das Falten der Linse erfolgt bei diesem System durch zwei separate Bauteile.

Um eine Intraokularlinse möglichst reproduzierbar und präzise in das Auge einbringen zu können, hat es sich gezeigt, dass nicht nur die Lage und Faltung der Linse als solches von Bedeutung ist sondern auch die Lage der Haptiken relativ zu der Linsenoptik beim Falten und beim Ausstoßen der Linse aus dem Injektor.

Um die Haptiken definiert positionieren zu können, ist in der genannten Patentanmeldung bereits ein Haptikschieber beschrieben, mit welchem, nachdem die Linsen gefaltet und in den Transportkanal eingebracht sind, die hintere Haptik in eine definierte Position zur Optik der Linse gebracht wird. Im Detail wird die hintere Haptik in einen hinteren Aufnahmebereich des Faltkörpers eingeführt. Weiterhin weist der Faltkörper einen Anschlag für eine vordere Haptik der Linse auf. Die Haptik wird dann mittels des Anschlags beim Anschieben auf die Linse gelegt. Beim weiteren Vorschub der Linse in dem Transportkanal wird die Linse gerollt.

Insbesondere sollen die Haptiken dabei möglichst in der Linsenoptik eingewickelt werden.

### Allgemeine Beschreibung der Erfindung

Vor dem vorstehend geschilderten Hintergrund liegt der vorliegenden Erfindung die Aufgabe zugrunde, einen Injektor für Intraokularlinsen bereitzustellen, der ein verbessertes Verhalten der Linse beim Falten und Ausstoßen der Linse aus dem Injektor ermöglicht.

Insbesondere sollen die Haptiken beim Ausstoßen der Linse aus dem Injektor bereits definiert in die Linsenoptik eingerollt sein, um ein möglichst sicheres und reproduzierbares Einbringen und Entfalten der Linse in dem Auge zu gewährleisten.

Gelöst werden diese Aufgaben bereits durch ein Magazin gemäß dem unabhängigen Patentanspruch 1. Vorteilhafte Ausführungsformen sind dem Gegenstand der Unteransprüche, der Beschreibung sowie den Figuren zu entnehmen.

Allgemein sieht die Erfindung vor, den im Stand der Technik beschriebenen Injektor derart weiterzuentwickeln, dass ein oder mehrere, vorzugsweise zwei, Haptikschieber direkt an dem Magazin bereitgestellt werden.

Im Detail sieht die vorliegende Erfindung ein Magazin für ein Injektorsystem zum Implantieren einer Linse in ein Auge vor, umfassend einen Aufnahmebereich für wenigstens eine Linse und zumindest einen bewegbaren Haptikschieber zum Verschieben einer Linsenhaptik in dem Magazin, vorzugsweise zum Schieben oder Aufschieben einer Linsenhaptik auf und/oder an eine Linsenoptik. Die auf- bzw. angeschobene Haptik und/oder Haptikspitze kann bzw. können in Kontakt mit der Linsenoptik oder einem Randbereich der Linse treten. Das Magazin ist insbesondere vorgesehen für ein Injektorsystem mit einem Faltkörper.

Durch den zumindest einen Haptikschieber kann eine Haptik in eine definierte Position überführt werden, so dass diese beim anschließenden, durch das Falten und den Vorschub induzierten Einrollen in die Linsenoptik und beim Entrollen im Auge eine definierte Position einnehmen kann.

Das Magazin stellt einen Bereich oder eine Linsenkammer dar zum Aufbewahren oder Lagern der Linse und gegebenenfalls zum Laden des Injektors. Der Aufnahmebereich für die Linse in dem Magazin zeichnet sich insbesondere dadurch aus, dass die Linse oder zumindest die Linsenoptik im Wesentlichen spannungsfrei, vorzugsweise eben, gelagert bzw. angeordnet werden kann. In einer Alternative kann die Linse in dem Aufnahmebereich auch bereits vorgefaltet positioniert sein.

Insbesondere umfasst der Haptikschieber einen äußeren Abschnitt, der an der Außenseite der Seitenwand des Magazins geführt ist, und einen inneren Abschnitt, der an der Innenseite der Seitenwand des Magazins geführt ist.

In einer Ausführungsform übernimmt der Haptikschieber nicht nur die Funktion des Verschiebens, sondern übernimmt auch die Funktion einer Halterung für die Linse. Das Magazin ist in dieser Ausführungsform dadurch gekennzeichnet, dass die Linse in dem Aufnahmebereich für die Linse mittels des Haptikschiebers festsetzbar oder festgesetzt ist. Insbesondere kann die Linse nicht einfach aus ihrer Position herausfallen.

Dazu sind das Magazin und der Haptikschieber vorzugsweise derart ausgeführt, dass der Haptikschieber, insbesondere der innere Abschnitt des Haptikschiebers, sowohl in seinem initialen Zustand als auch in seinem verschobenen Zustand wenigstens einen Bereich der Linse in dem Aufnahmebereich festsetzt. Beispielsweise ist ein Randbereich der Linsenoptik von dem Haptikschieber überdeckt.

Vorzugsweise stellt der Haptikschieber einen Mitnehmer für eine Haptikspitze einer Linsenhaptik bereit, um die Haptik verschieben zu können. In einer Ausgestaltung des Magazins wird der Mitnehmer durch eine Aussparung in dem inneren Abschnitt des Haptikschiebers bereitgestellt.

Der Haptikschieber ist insbesondere beweglich an dem Magazin, vorzugsweise mittels einer Rastverbindung, montiert. In einer Ausführungsform ist der Haptikschieber auf einer Seitenwand und/oder auf dem Boden des Magazins im Wesentlichen axial verschiebbar angeordnet, vorzugsweise aufgesteckt bzw. eingesteckt. In einer Ausgestaltung geht der äußere Abschnitt des Haptikschiebers mit der Außenseite der Seitenwand des Magazins eine Rastverbindung ein. In einer weiteren Ausführungsform ist der Haptikschieber an einer Seitenwand des Magazins montiert und erstreckt sich durch eine Öffnung in der Seitenwand des Magazins in das Magazin hinein.

Vorzugsweise wird der Kopfabschnitt oder ein äußerer Abschnitt des Haptikschiebers auf der Oberseite der Seitenwand bzw. direkt an der Seitenwand des Magazins geführt. Um den Haptikschieber besser handhaben zu können, kann auf dem Kopfabschnitt des Haptikschiebers bzw. auf dem äußeren Abschnitt des Haptikschiebers, der sich durch die Seitenwand erstreckt, eine Erhebung zum Verschieben ausgebildet sein. Diese dient als Anlagefläche für einen Finger.

In einer Ausgestaltung besitzt eine Intraokularlinse zwei Haptiken. Vorzugsweise sind daher auch zwei Haptikschieber vorgesehen. Vorzugsweise sind die beiden Haptikschieber in entgegengesetzten Richtungen bewegbar.

Um ein zuverlässiges und sicheres Einbringen der Linse in das Auge zu unterstützen, sollten die Haptiken nicht von der Linse abstehen. Die Haptiken sollten sich vielmehr in einer definierten Position befinden. Sie können bzw. sollen zum Beispiel in die Linse eingerollt sein oder werden. In Abhängigkeit von der Ausrichtung der Linse im Injektor liegen zum Beispiel eine Haptik im vorderen Bereich (die vordere Haptik) und eine Haptik im hinteren Bereich (die hintere Haptik) des Magazins.

In einer Ausführungsform weist das Magazin dazu einen hinteren Haptikschieber, welcher einer hinteren Haptik zugeordnet ist, und einen vorderen Haptikschieber, welcher einer vorderen Haptik zugeordnet ist, auf. Der hintere Haptikschieber ist zum Schieben oder Aufschieben der hinteren Haptik, vorzugsweise auf und/oder an die Optik der Linse, in Richtung der Vorderseite des Injektors verschiebbar angeordnet. Der vordere Haptikschieber ist zum Schieben oder Aufschieben der vorderen Haptik, vorzugsweise auf und/oder an die Linsenoptik, in Richtung der Rückseite des Injektors verschiebbar angeordnet.

Die beiden Haptikschieber können beispielsweise einzeln nacheinander oder auch gleichzeitig betätigt werden. In einer alternativen Variante sind der vordere und der hintere Haptikschieber derart miteinander gekoppelt, dass durch eine einzige Betätigung sowohl die vordere Haptik als auch die hintere Haptik verschiebbar, vorzugsweise auf und/oder an die Linsenoptik schiebbar, sind.

In einer ersten Ausführungsform einer gekoppelten Bewegung der Haptikschieber umfasst das Magazin einen beweglich, vorzugsweise drehbar, gelagerten Verbindungsabschnitt. Der Verbindungsabschnitt ist jeweils über ein Drehgelenk mit dem vorderen Haptikschieber und dem hinteren Haptikschieber verbunden. Insbesondere durch ein Aufschieben des Magazins auf die Kanüle des Injektors wird die gekoppelte Bewegung induziert. Der Verbindungsabschnitt wird auf einem hinteren Abschnitt der Kanüle vollständig zur Anlage gebracht. Dies führt zu einer Drehung des Verbindungsabschnitts, welches wiederum in eine gegenläufige Bewegung der beiden Schieber resultiert.

In einer zweiten Ausführungsform einer gekoppelten Bewegung umfasst das Magazin einen axial verschiebbaren Schlitten, in dem der Aufnahmebereich für die Linse angeordnet ist. Der Schlitten wird durch eine axiale Verschiebung des vorderen Haptikschiebers in Richtung des hinteren Haptikschiebers verschoben. Zunächst verschiebt der vordere Haptikschieber die vordere Haptik in Richtung der Linsenoptik. Der Haptikschieber kommt dabei zur Anlage an dem Schlitten und verschiebt diesen in Richtung des hinteren Haptikschiebers. Die Linse wird dadurch in Richtung des hinteren Haptikschiebers verschoben. Dadurch wird auch die hintere Linsenhaptik an die Optik der Linse herangeführt. Der hintere Haptikschiebers selbst wird dabei nicht bewegt.

Das Magazin ist in einer weiteren Ausführungsform dadurch gekennzeichnet, dass an einem Abschnitt ,vorzugsweise an der Innenseite, der beiden Seitenwände des Magazins jeweils eine Rampe angeordnet ist. Vorzugsweise ist für die vordere Haptik eine von der Vorderseite zur Rückseite des Magazins ansteigende Rampe vorgesehen. Vorzugsweise ist für die hintere Haptik eine von der Rückseite zur Vorderseite des Magazins ansteigende Rampe vorgesehen. Die Rampen müssen sich hierbei nicht vollständig durch das Magazin erstrecken. In einer weiteren Ausführungsform ist an der Innenseite der beiden Seitenwände des Magazins jeweils eine von der Vorderseite des Magazins zunächst ansteigende und dann in Richtung der Rückseite des Magazins wieder abfallende Rampe bereitgestellt. Vorzugsweise wird der Aufnahmebereich für die wenigstens eine Linse jeweils durch eine, vorzugsweise in etwa mittige, Aussparung in der Rampe bereitgestellt. Die Haptikspitze der vorderen Linsenhaptik und die Haptikspitze der hinteren Linsenhaptik liegen in dem initialen, nicht verschobenen Zustand jeweils neben dem Aufnahmebereich auf der Rampe auf. Dadurch kann die Linse insgesamt möglichst spannungsfrei gelagert werden. Alternativ ist auch eine Vorspannung der Linse, beispielsweise durch eine Vorfaltung der Linse, möglich.

In dem Aufnahmebereich für die Linse sind in einer weiteren Ausführungsform jeweils zumindest zwei Köpfe angeordnet, auf denen jeweils ein Randbereich der Linsenoptik aufliegen kann. Dadurch kann die Kontaktfläche zwischen dem Magazin und der Linse verringert werden

Im Bereich der Erfindung liegt auch ein Injektorsystem für das vorstehend beschriebene Magazin bzw. welches dieses beinhaltet. Es ist ein Injektorsystem zum Implantieren einer Linse in ein Auge umfassend:
- einen Injektorkörper mit einer Vorderseite und einer Rückseite,
- eine an der Vorderseite des Injektorkörpers angeordnete Kanüle, die einen Transportkanal für eine zu implantierende Linse bereitstellt, wobei dem Transportkanal eine Linse über eine vorzugsweise seitliche Eingangsöffnung zuführbar ist,
- eine Ausführungsform des vorstehend und nachfolgend beschriebenen Magazins mit einem Aufnahmebereich für wenigstens eine Linse, wobei das Magazin so angeordnet ist, dass dem Transportkanal eine Linse über die vorzugsweise seitliche Eingangsöffnung zuführbar ist,
- einen in das Magazin und die Eingangsöffnung einfügbaren Faltkörper zum Einschieben der Linse in den Transportkanal derart, dass die Linse zumindest abschnittsweise um den Faltkörper faltbar ist, und
- einen Schieber, welcher im Injektorkörper, vorzugsweise axial, verschiebbar angeordnet ist und über die Vorderseite des Injektorkörpers so in den Transportkanal einschiebbar ist, dass die Linse aus dem Transportkanal ausgeschoben werden kann.

In einer Ausführungsform ist das Magazin zum Laden des Injektorsystems mit einer Linse mit der Kanüle verbindbar ist, vorzugsweise auf die Kanüle des Injektors so aufschiebbar oder aufsteckbar, dass die Kanüle zumindest abschnittsweise im Inneren des Magazins oder am Magazin angeordnet ist.

In einer bevorzugten Ausgestaltung der Erfindung wird das Magazin als ein separates Modul bereitgestellt. Das Magazin kann hierbei werksseitig bereits an dem Injektor montiert sein oder erst durch den Anwender befestigt werden.

Vorzugsweise weist das Magazin einen Austrittsbereich bzw. eine Ausgangsöffnung auf, der bzw. die so ausgerichtet zu der Eingangsöffnung in dem Transportkanal angeordnet ist, dass die Linse mittels des Faltkörpers aus dem Magazin über Austrittsbereich bzw. die Ausgangsöffnung in den Transportkanal einschiebbar ist. In dieser Variante kann die Kanüle direkt in das in einem vorzugsweise separaten Behältnis befindliche Magazin eingeführt werden und zum Beispiel mit oder in diesem verrasten, so dass eine Funktionseinheit hergestellt wird. Vorzugsweise wird bzw. werden das Magazin und/oder die Linse in dem Behältnis steril gelagert.

Das erfindungsgemäße Magazin und/oder das Injektorsystem ist bzw. sind insbesondere geeignet für alle weichen, faltbaren Intraokularlinsen. Diese sind zum Beispiel aufgebaut aus Acryl, Silikon und/oder Hydrogel. Das erfindungsgemäße Magazin ist leicht anpassbar an unterschiedliche Linsentypen, insbesondere hinsichtlich deren Geometrie/Design und/oder deren Material.

Die konkreten Abmessungen und/oder Formen der einzelnen Module und/oder der Merkmale des Magazins und/oder des Injektorsystems sind unter anderem abhängig von dem Design einer zu implantierenden Intraokularlinse. Das erfindungsgemäße Magazin und/oder der Injektor kann bzw. können als vorgeladener Wegwerf- oder Einweg-Injektor verwendet werden.

Die vorliegende Erfindung wird anhand der nachfolgenden Ausführungsbeispiele im Einzelnen erläutert. Hierzu wird auf die beigefügten Zeichnungen Bezug genommen. Gleiche Bezugszeichen in den einzelnen Zeichnungen beziehen sich auf die gleichen Teile.
- Fig. 1a. und 1.b: zeigen den erfindungsgemäßen Injektor in seinem zusammengebauten Zustand (mit noch nicht eingeführtem Faltkörper) in einer perspektivischen Ansicht (Fig. 1.a) und in einer Seitenansicht (Fig. 1.b).
- Fig. 2.a bis 2.c: illustrieren in einer perspektivischen Ansicht den vorderen Abschnitt des Injektors mit der Kanüle und dem auf der Kanüle aufgesteckten Magazin mit noch nicht eingeführtem Faltkörper (Fig. 2.a), mit eingeführtem Faltkörper (Fig. 2.b) und noch einmal ohne Faltkörper (Fig. 2.c).
- Fig. 3.a bis 3.c: illustrieren in einer perspektivischen Ansicht das Magazin gemäß einer ersten Ausführungsform in verschiedenen Zuständen: mit den seitlich angebrachten Haptikschiebern (Fig. 3.a), ohne Haptikschieber (Fig. 3.b) und ohne Linse (Fig. 3.c).
- Fig. 4.a bis 4.c: illustrieren einen Haptikschieber in einer Seitenansicht auf seine Innenseite (Fig. 4.a), in einer Seitenansicht auf seine Außenseite (Fig. 4.b) sowie in einer perspektivischen Ansicht auf die Unterseite seines Kopfteils (Fig. 4.c).
- Fig. 5.a bis 5.c: zeigen in einer perspektivischen Ansicht die Arbeitsschritte bei der Vorbereitung des Injektors zum Einführen der Linse in das Auge: das Magazin in seinem Ausgangszustand (Fig. 5.a), mit aufgeschobener hinterer Haptik (Fig. 5.b) und mit aufgeschobener vorderer Haptik (Fig. 5.c).
- Fig. 6.a bis 6.f: zeigen eine zweite Ausführungsform eines Magazins mit zwei seitlich an den Magazinwänden angebrachten Haptikschiebern, die sich durch die Magazinwand erstrecken: in zwei perspektivischen Ansichten seitlich von oben (Fig. 6.a und 6.b), in einer Aufsicht (Fig. 6.c), in einer Aufsicht mit durchscheinenden Wänden (Fig. 6.d) sowie in einer Seitenansicht mit durchscheinenden Wänden (Fig. 6.e und 6.f).
- Fig. 7.a bis 7.e: zeigen in einer perspektivischen Ansicht eine dritte Ausführungsform eines Magazins mit zwei Haptikschiebern, deren Bewegung miteinander gekoppelt ist: das Magazin ohne Haptikschieber (Fig. 7.a), mit eingesetzten Haptikschiebern in ihrem Ausgangszustand (Fig. 7.b) und in ihrem Endzustand mit an der Linse angelegten Haptiken (Fig. 7.c) sowie mit dargestellter Kanüle (Fig. 7.d und 7.e).
- Fig. 8.a und 8.b: zeigen in einer perspektivischen Ansicht eine vierte Ausführungsform eines Magazins mit zwei Haptikschiebern, deren Bewegung wiederum gekoppelt ist: das Magazin im Ausgangszustand (Fig. 8.a) und im Endzustand mit an der Linse angelegten Haptiken (Fig. 8.b).

### Detaillierte Beschreibung der Erfindung:

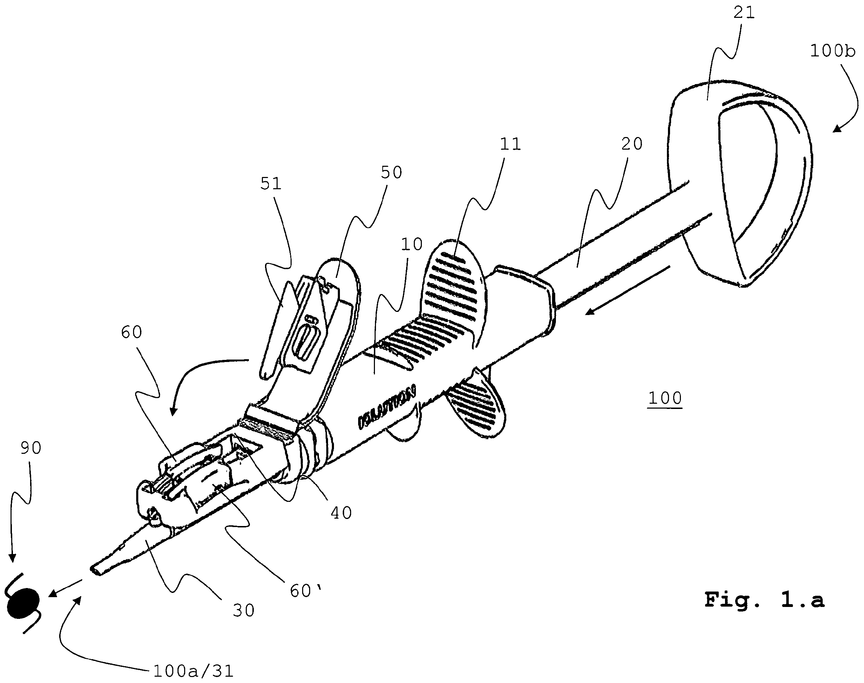

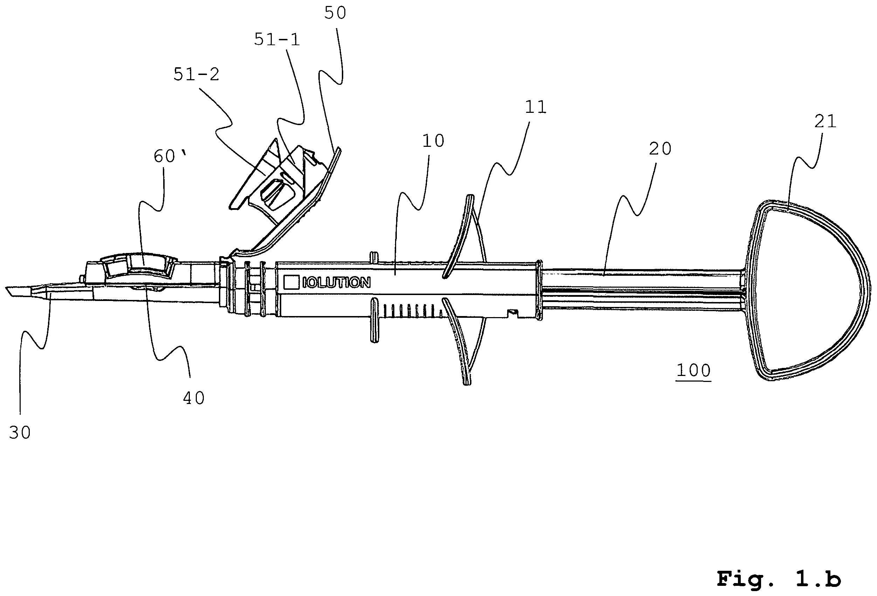

Das erfindungsgemäße Magazin wird am Beispiel eines modular aufgebauten Injektorsystems 100 beschrieben. Figur 1 zeigt dazu einen Injektor 100 in seinem zusammengebauten Zustand. Die Module des Injektors 100 umfassen ein Gehäuse 10, einen Schieber 20, eine Kanüle 30 sowie das erfindungsgemäße Magazin 40.

Die Kanüle 30 ist an der Vorderseite 10a des Gehäuses 10 angeordnet. Die Kanüle 30 und das Gehäuse 10 sind zum Beispiel einrastend miteinander verbunden. Die Kanüle 30 hat einen Transportkanal 31 für die Linse 90 zum Ausbringen der Linse 90 aus dem Injektor 100 bzw. zum Einbringen der Linse 90 in ein Auge. Auf der Kanüle 30 ist das Magazin 40 positioniert, vorzugsweise aufgesteckt. Vorzugsweise sind die Kanüle 30 und das Magazin 40 einrastend miteinander verbunden.

Die Faltklappe 50, welche die Faltrippe 51 trägt, befindet sich im noch nicht heruntergeklappten Zustand. Die Linse 90 befindet sich dadurch noch nicht im Transportkanal 31 der Kanüle 30. Der Injektor 100 ist sozusagen noch gesichert.

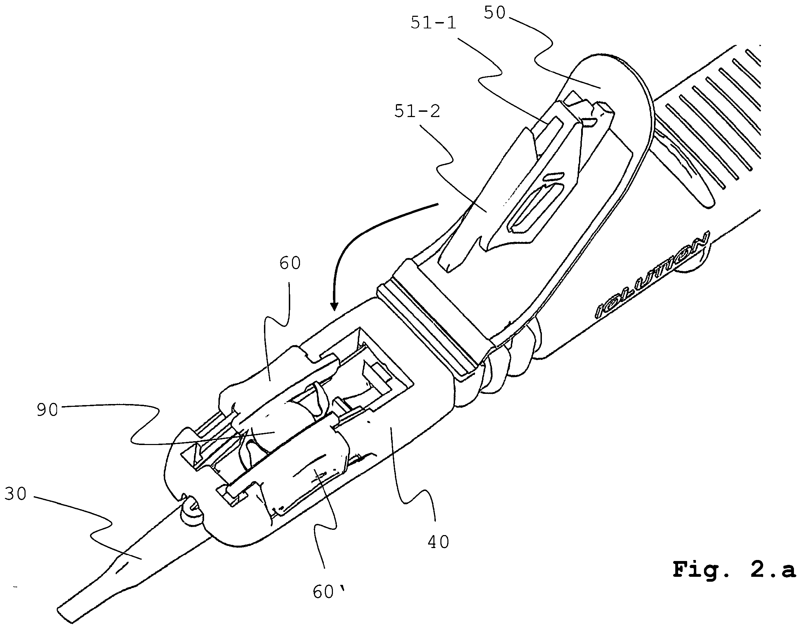

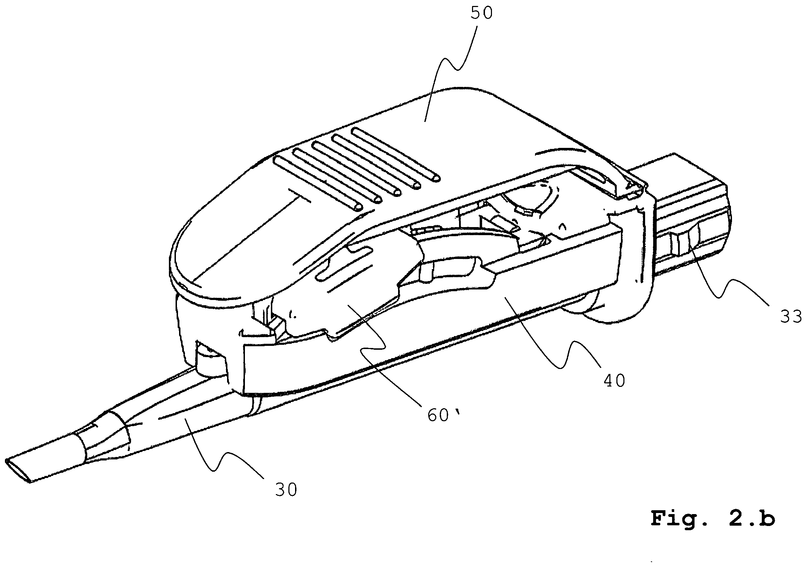

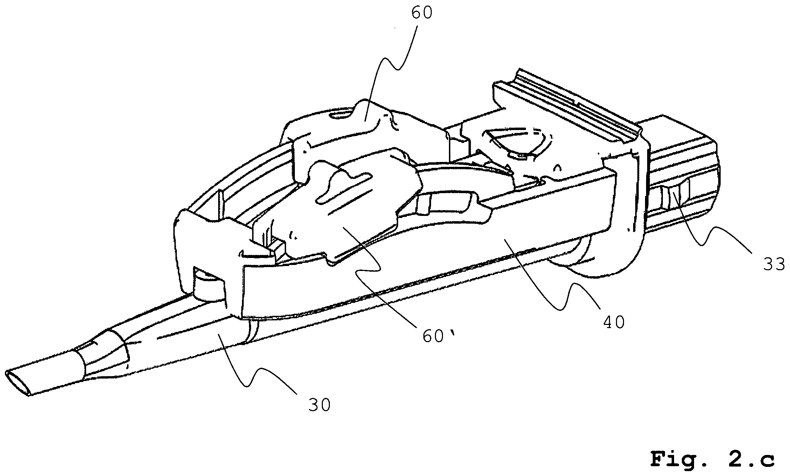

Durch ein Herunterklappen der Faltklappe (siehe dazu die Figur 2.b) wird die Linse 90 in den Transportkanal 31 der Kanüle 30 überführt. Der Injektor 100 ist dann sozusagen entsichert. Der Injektor 100 ist betriebsbereit. Der Schieber 20 wird über die Rückseite 10b des Gehäuses 10 eingeführt. Er ist axial verschiebbar in dem Gehäuse 10 angeordnet. Der Schieber 10 ist in dem Gehäuse 10 so angeordnet, dass er aus der Vorderseite 10a des Gehäuses 10 so austritt, dass er in den Transportkanal 31 der Kanüle 30 eintreten kann. Der Schieber 10 und der Transportkanal 31 sind so in ihrer Form und/oder ihrer Größe aufeinander angepasst, dass der Schieber 20 auch in dem Transportkanal 31 axial verschiebbar angeordnet ist. Über ein Verschieben bzw. Drücken des Schiebers 20 in Richtung der Vorderseite 100a des Injektors 100 wird die Linse 90, welche sich im Transportkanal 31 der Kanüle 30 befindet, aus dem Injektor 100 ausgestoßen.

Die Vorderseite 30a der Kanüle 30 oder des Transportkanals 31 definiert die Austrittsöffnung für die Linse 90. Der Schieber 20 bzw. der Injektor 100 kann beispielsweise betätigt werden, indem der Zeigefinger und der Mittelfinger an den Griffen 11 des Gehäuses 10 anliegen und der Daumen in den Griff 21 des Schiebers 20 eingreift.

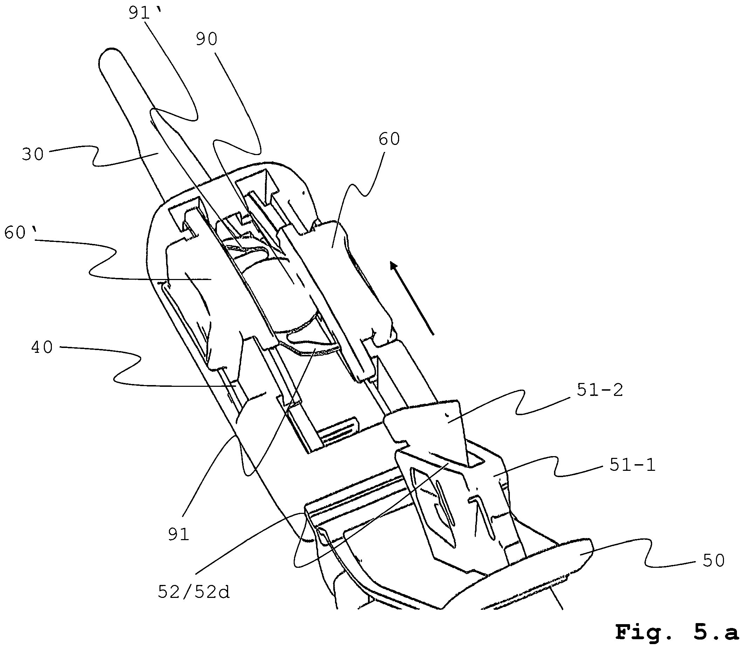

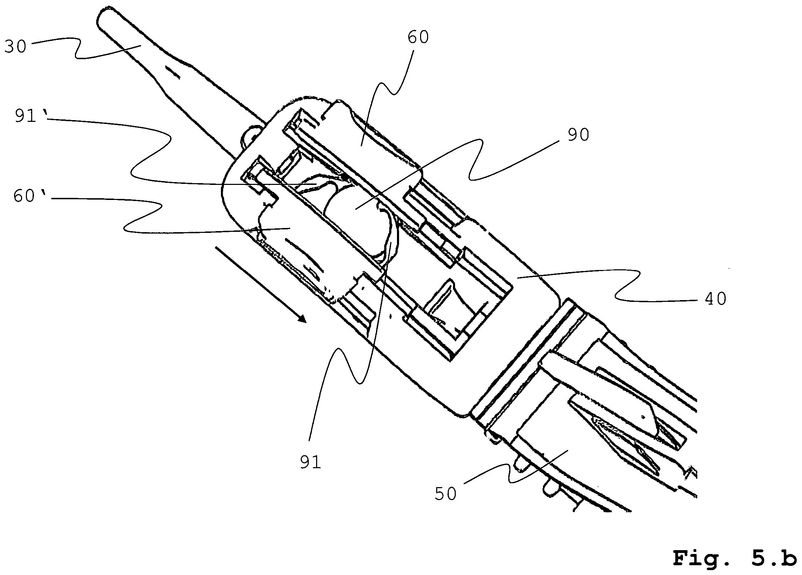

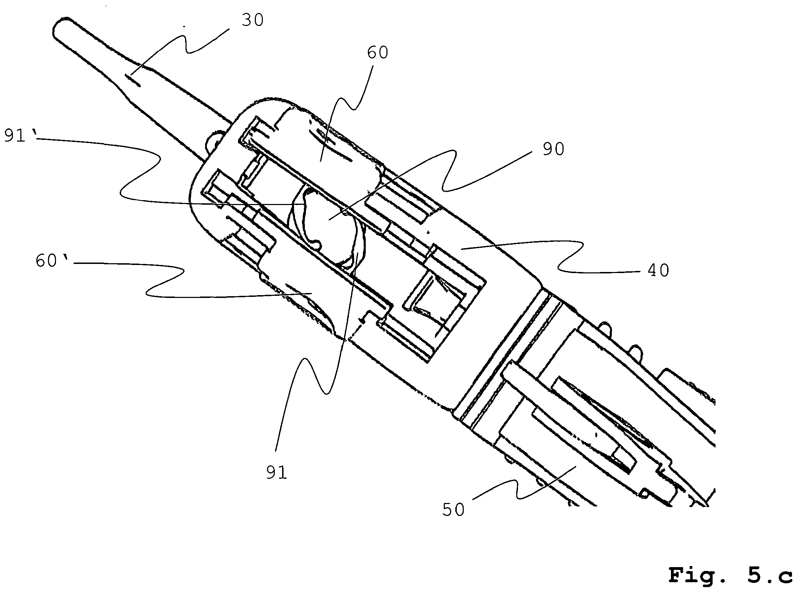

Für weitere Details zum Laden bzw. Bestücken, Sichern, Entsichern und/oder Anwenden des Injektors 100 bzw. Ausstoßen der Linse sei auf die Ausführungen zu den Figuren 5.a bis 5.c und die WO 2012/155887 A1 verwiesen. In dem gezeigten Beispiel ist das Magazin 40 bereits, insbesondere werksseitig, an dem Injektor 100 vormontiert

Die Figuren 2.a und 2.b zeigen eine Detailansicht des vorderen Abschnitts des Injektors 100. Dargestellt sind die Kanüle 30 und das auf die Kanüle 30 aufgesteckte Magazin 40. Die Figur 2.a zeigt dabei direkt den Injektor 100 aus den Figuren 1.a und 1.b. Das Magazin 40 ist auf der Kanüle 30, vorzugsweise einrastend, befestigt, insbesondere aufgesteckt. In dem Magazin 40 ist die Linse 90 angeordnet. Der Faltkörper 50 zum Einbringen der Linse 90 in den Transportkanal 31 der Kanüle 30 ist an dem Injektorkörper 10 befestigt. Der Faltkörper 50 ist hier zweiteilig aufgebaut, durch einen Sockel 51-1 und eine in dem Sockel 51-2 verschiebbare Faltrippe 51-2. Weitere Details hierzu sind in der nachfolgenden Beschreibung zur Figur 5.c zu finden.

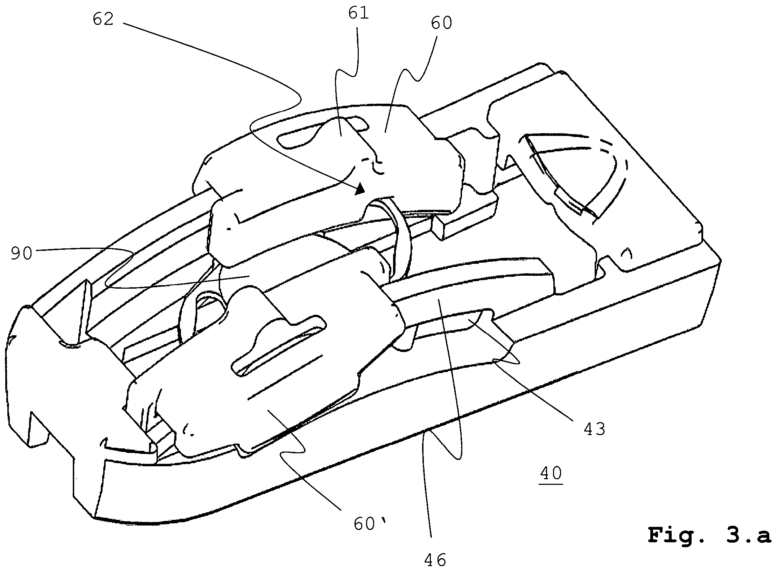

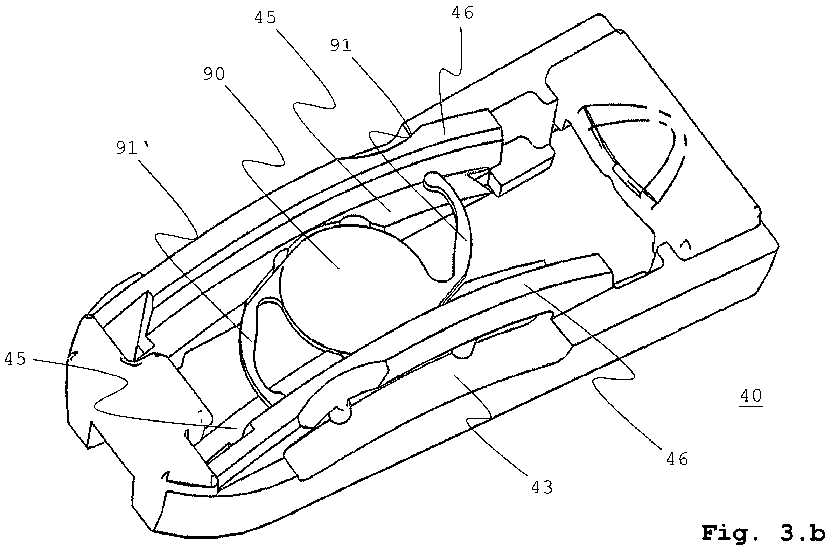

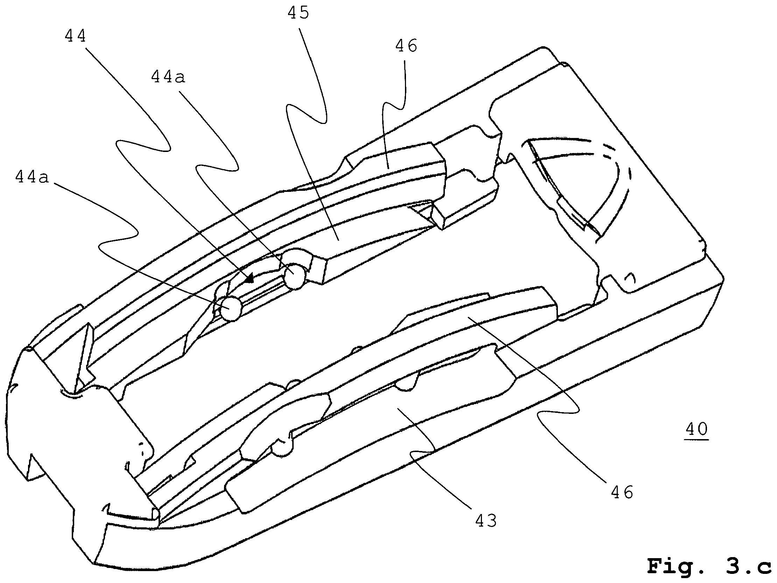

Die Figuren 2.b und 2.c (als auch die Figuren 3.a bis 4.c) zeigen eine gegenüber dem in Figur 2.a dargestellten Magazin 40 leicht veränderte Variante. Die Haptikschieber 60 und 60' besitzen jeweils an ihrer Oberseite einen Vorsprung 61, der insbesondere als Anlagefläche für einen Finger beim Verschieben dienen soll. Figur 2.b zeigt den Zustand, in dem der Faltkörper 50 nach unten geklappt und die Linse 90 in den Transportkanal 31 der Kanüle 30 überführt und dabei im wesentlichen u-förmig um den Faltkörper 50 gefaltet wurde. Es sei hier angemerkt, dass die Haptikschieber 60 und 60' in der Figur 2.b nicht verschoben dargestellt sind. Figur 2.c zeigt noch einmal die Darstellung aus Figur 2.b ohne den Faltkörper 50. Die Kanüle 30 ist mittels einer Rastverbindung 33 an dem Injektorkörper 10 montiert.

Die Figuren 3.a bis 3.d illustrieren nun das Magazin 40 als solches. Um die Details des Magazins 40 besser darstellen und erläutern zu können, sind in den einzelnen Figuren zum Teil einzelne Komponenten entfernt. Die beiden Haptiken 91 und 92 der Linse 90 sind jeweils noch nicht auf die Linse 90 bzw. auf die Linsenoptik 90 aufgeschoben. Die beiden Haptikschieber 60 und 60' befinden sich somit noch in ihrem Ausgangszustand.

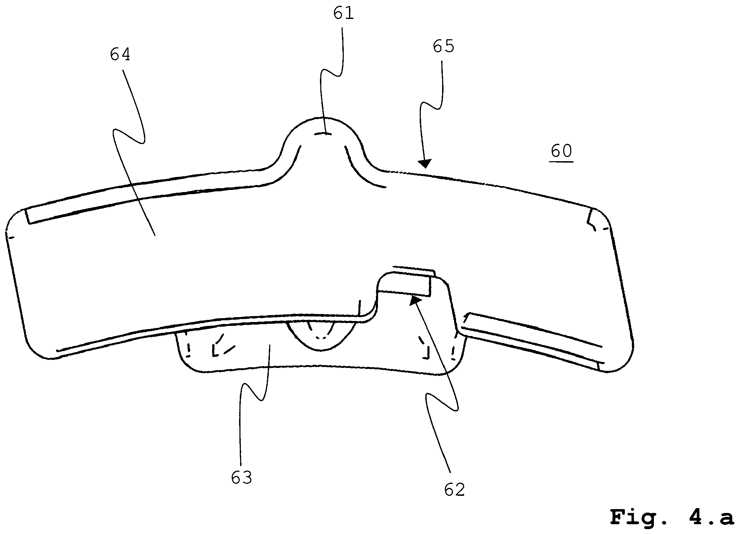

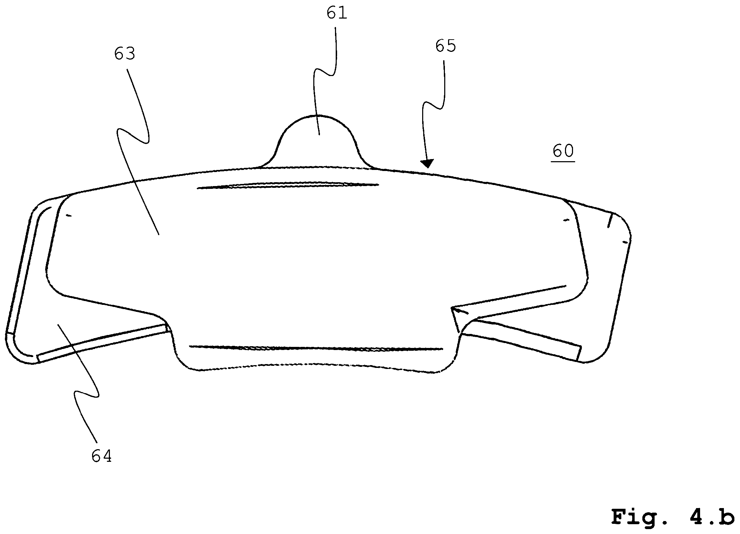

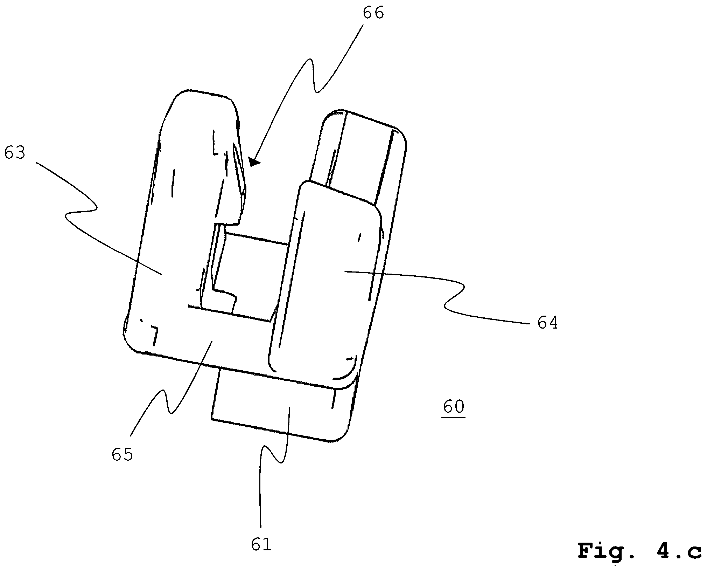

Zunächst ist in der Figur 3.a das vollständige Magazin 40 mit den beiden aufgebrachten Haptikschiebern 60 und 60' dargestellt. Die Erläuterungen zum Aufbau und der Funktion erfolgen an nur einem Haptikschieber 60, sind aber für den anderen Haptikschieber 60' zutreffend (siehe dazu auch die Figuren 4.a bis 4.c). Der Haptikschieber 60 ist auf der Seitenwand 43 des Magazins 40 angeordnet. Er ist auf der Seitenwand 43, vorzugsweise axial, verschiebbar befestigt. Dazu ist an der Oberseite der Seitenwand 43 eine Schiene 46 ausgebildet, auf welcher der Haptikschieber 60 gleiten kann.

Der Haptikschieber 60 weist an seinem inneren Abschnitt 64 eine Aussparung 62 auf. Diese Aussparung 62 stellt einen Aufnahmebereich für die Haptikspitze der hier hinteren Haptik 91 dar. Im Lagerzustand des Magazins 40 ist dort die Haptikspitze sicher positioniert. Die Aussparung 62 stellt aber auch einen Mitnehmer für die Haptikspitze beim Verschieben des Haptikschiebers 60 dar (siehe dazu die Beschreibung zu den Figuren 5.a bis 5.c).

In Figur 3.b ist das Magazin 40 nun mit nicht montierten Haptikschiebern 60 und 60' dargestellt. Figur 3.c zeigt die gleiche Darstellung nur mit entfernter Linse 90. In dem Magazin 40 ist an der Innenseite der Seitenwand 43 jeweils eine Art Rampe 45 ausgebildet, die vorzugsweise zumindest abschnittsweise gekrümmt ist. Die Rampe 45 ist zunächst von der Vorderseite 100a des Injektors 100 ansteigend und dann in etwa ab dem mittleren Bereich wieder abfallend. In etwa mittig ist in der Rampe 45 der Aufnahmebereich 44 für die Linse 90 angeordnet. Der Aufnahmebereich 44 ist hier als eine Aussparung in der Rampe 45 ausgeführt. Die Gestalt oder die Kontur des Aufnahmebereichs 44 ist im Wesentlichen an die Kontur der Linse 90 angepasst. Vorzugsweise ist die Kontur des Aufnahmebereichs zumindest abschnittsweise gekrümmt. Die Linse 90 liegt mit dem Haptikansatz der hier vorderen Haptik 91' und einem Randbereich der Linsenoptik 90 auf bzw. in dem Aufnahmebereich 44 für die Linse 90. Dagegen liegt die Haptikspitze der hier hinteren Haptik 91 nicht in dem Aufnahmebereich 44. Die Haptikspitze liegt hier neben dem Aufnahmebereich 44 auf dem abfallenden Bereich der Rampe 45. Das gleiche gilt auch für die andere Seite der Linse 90 und die hier nur teilweise zu sehende rechts dargestellte Rampe 45. Dadurch können die beiden Haptiken 91 und 91' und die Linsenoptik 90 im Wesentlichen auf einer Ebene liegen und dadurch möglichst spannungsfrei gelagert werden. Zudem sind die beiden Haptiken 91 und 91' dadurch, dass sie auf der Rampe 45 aufliegen, verschiebbar. Im Aufnahmebereich 44 sind noch zwei, hier entlang der Längsachse, verteilt Köpfe 44a angeordnet. Dadurch kann die Linsenoptik 90 insbesondere auf einer geringen Fläche gelagert werden.

Die beiden Haptikschieber 60 und 60' übernehmen aber nicht nur die Funktion des Verschiebens der Haptiken 91, 91' oder des Aufschiebens der Haptiken 91 und 91' auf die Linse 90. Sie übernehmen auch die Funktion einer Halterung für die Linse 90 in dem Magazin 40 bzw. in dem Aufnahmebereich 44 für die Linse 90. Der Haptikschieber 60 überdeckt mit seinem inneren Abschnitt 64 sowohl in seinem Ausgangszustand als auch in seinem verschobenen Endzustand den Aufnahmebereich 44 für die Linse 90 (siehe dazu die Figuren 3.a und 5.a bis 5.c).

Die Figuren 4.a bis 4.c zeigen nun den Haptikschieber 60 und 60' als solches. Figur 4.a zeigt zunächst eine Ansicht auf den inneren Abschnitt 64 des Haptikschiebers 60. Dieser ist hier, insbesondere an seiner Unterseite, gekrümmt ausgeführt, damit er auf der ebenfalls gekrümmten Rampe 45 entsprechend gleitend kann. In der Unterseite des inneren Abschnitts 64 ist die Aussparung 62 zur Aufnahme und zur Mitnahme der Haptikspitze der Haptiken 91, 91' eingebracht. Auf dem Kopfabschnitt 65 des Haptikschiebers 60 ist ein Vorsprung 61 vorhanden, welcher beispielsweise beim Verschieben des Haptikschiebers 60 eine Anlagefläche für einen Finger bereitstellt.

Die Figur 4.b zeigt eine Ansicht auf den äußeren Abschnitt 63 des Haptikschiebers 60. Um auch den inneren Aufbau des Haptikschiebers 60 besser darstellen zu können, zeigt Figur 4.c den Haptikschieber 60 in einer auf den "Kopf" gedrehten Ansicht mit dem äußeren Abschnitt 63, dem inneren Abschnitt 64 und dem verbindenden Kopfteil 65. Der Haptikschieber 60 wird im Wesentlichen über den äußeren Abschnitt 63 an dem Magazin 40 befestigt. Der äußere Abschnitt 63 besitzt dazu auf seiner Innenseite einen Vorsprung 66. Der Vorsprung 66 hintergreift die an der Oberseite der Magazinseitenwand 43 geformte Schiene 46. Die Schiene 46 wird dabei zwischen der Unterseite des Kopfteils 65 und dem Vorsprung 66 des äußeren Abschnitts 63 axial verschiebbar befestigt oder festgesetzt. Die Innenseite des inneren Abschnitts 64 des Haptikschiebers 60 ist dagegen eben ausgebildet. Sie wird auf der Innenseite der Magazinseitenwand 43 geführt.

Die Figuren 5.a bis 5.c illustrieren die Arbeitsschritte bei der Vorbereitung des Injektors 100 zum Einführen der Linse 90 in das Auge.

Die Figur 5.a zeigt den Injektor 100 mit dem vorzugsweise vormontierten Magazin 40, beispielsweise nach der Entnahme aus der Verpackung. Die Linsenhaptiken 91 und 91' sowie die Haptikschieber 60 und 60' befinden sich jeweils noch in ihrem Ausgangszustand. Es wird bespielhaft mit dem hier rechts dargestellten Haptikschieber 60, der der hinteren Haptik 91 zugeordnet ist, gestartet. Der Haptikschieber 60 wird dazu in Richtung der Vorderseite 100a des Injektors 100 geschoben (in Pfeilrichtung). Dieser nimmt dabei die Haptik 91 bzw. ihre Haptikspitze soweit mit, dass diese vorzugsweise abschnittsweise oder vollständig auf einem Seitenbereich der Linsenoptik 90 zur Auflage und/oder Anlage kommt. Die Haptik 91 kann dabei Kontakt mit der Linsenoptik haben. Sie kann aber auch oberhalb der Linse 90 beabstandet von dieser sein. Die Haptikspitze kann dabei auch aus der Aussparung 62 im Haptikschieber 60 herausgeschoben werden, so dass sie nur an der Innenseite des Haptikschiebers 60 anliegt. Der Haptikschieber 60 kann in dieser Endposition zum Beispiel mit dem Magazin 40 verrasten. Der nach vorne geschobene Haptikschieber 60 und die ebenso nach vorne geschobene und aufgelegte Haptikspitze der hinteren Haptik sind in der Figur 5.b dargestellt.

Der prinzipiell gleiche Vorgang wird nun für die vordere Haptik 90' durchgeführt. Dazu wird der vordere, hier links dargestellte Haptikschieber 60' in Richtung der Rückseite 100b des Injektors 100 geschoben (Pfeilrichtung in Figur 5.b). Er nimmt dabei die Haptikspitze soweit mit, dass sie vorzugsweise auf dem Seitenbereich der Linsenoptik 90 zur Auflage kommt. Die Haptikspitze kann dabei auch wieder aus der Aussparung 62 in dem Haptikschieber 60' herausgeschoben werden, so dass sie nur noch an der Innenseite des Haptikschiebers 60' anliegt. Der Haptikschieber 60' kann in dieser Endposition wiederum zum Beispiel mit dem Magazin 40 verrasten. Der nach hinten geschobene Haptikschieber 60' und die aufgelegte Haptikspitze der vorderen Haptik 91' ist in der Figur 5.c dargestellt.

Die Linse 90 ist nun so konfiguriert, dass sie gefaltet werden kann. Die Linse 90 wird hierbei durch den herunter geklappten und in das Magazin 40 eingeführten Faltkörper 50 gefaltet (siehe dazu z.B. Figur 2.b) und dabei auch in den Transportkanal 31 des Kanüle 30 eingeschoben. Der Schieber 20 befördert dann die Linse 90 in Richtung der Vorderseite 100a des Injektors 100 und aus diesen heraus in das Auge (nicht in den Figuren dargestellt). Beim Schieben bzw. Ausstoßen der Linse 90 wird diese gerollt. Die Haptiken 91 und 91' sind durch das Verschieben insbesondere derart angeordnet, dass sie beim Faltvorgang als auch nach dem Falten so definiert positioniert sind, dass sie beim Vorgang des Ausstoßens der Linse 90 in die Linse 90 eingerollt werden können. Beispielsweise können die Haptiken 91 und 91' dadurch das Einbringen der Linse 90 in das Auge nicht behindern. Nachdem die Linse 90 in das Auge eingeführt wurde, entrollt sich diese wieder und die Haptiken 90 und 91' nehmen ihre Positionen ein.

Die Arbeitsweise des Injektors 100 ist nachfolgend noch einmal beschrieben, in den Figuren aber nicht dargestellt (siehe dazu insbesondere die WO 2012/155887 A1). In einem nächsten Schritt wird der Injektor 100 entsichert. Dazu wird die Linse 90 von ihrer Ausgangsposition in dem Magazin 40 in ihre Transportposition in der Kanüle 30 bzw. im Transportkanal 31 bewegt bzw. überführt. Die genannte Transportposition kennzeichnet die Position, von der aus die Linse 90 mittels des Schiebers 20 aus dem Injektor 100 ausgestoßen und in ein Auge eingeführt werden kann. Dazu wird die Faltklappe 50 in Richtung auf die Oberseite des Magazins 40 geklappt oder geschwenkt. Die an der Unterseite der Faltklappe 50 angeordnete Faltrippe 51 greift zwischen den beiden Haptikschiebern 60 und 60' hindurch in das Magazin 40 ein. Die Faltklappe 51 kommt in Auflage auf die Oberseite der Linse 90 und schiebt sie, indem die Linse 90 im Querschnitt U-förmig gefaltet wird, aus ihrer Ausgangsposition im Magazin 40 heraus in den Transportkanal 31 der Kanüle 30 hinein. Um ein sicheres Falten der Linse 90 und ein mögliches Verrutschen und Verklemmen der Linse 90 zu verhindern, sind an der Faltrippe 51 vorzugsweise zwei Rückhaltekanten 52 vorgesehen. Der Faltkörper 51 ist hier zweiteilig aufgebaut. Er wird durch einen Sockel 51-1 bereitgestellt, in welchem das eigentliche Faltelement 51-2 eingesteckt ist. Die Rückhaltekanten 52 werden vom Sockel 51-1 bereitgestellt. Das Faltelement 51-2 ist in dem Sockel 51-1 in Richtung zur Oberseite des Faltkörpers 51 verschiebbar angeordnet. Ohne die gebildeten Rückhaltkanten 52 könnte die Linse 90 seitlich nach oben wegrutschen und verklemmen.

In einem nächsten Schritt wird die Linse 90 aus dem Injektor 100 ausgebracht und in ein Auge eingeführt. Mittels des Schiebers 20 wird die Linse 90 aus dem Injektor 100 ausgeschoben. Bei einem weiteren Vorschieben, welches zu einem Aufrollen der Linse 90 führt, werden die beiden Haptik en 91 und 91' in die Linse 90 eingerollt. Sie besitzen somit beim Auswerfen der Linse 90 eine definierte Position.

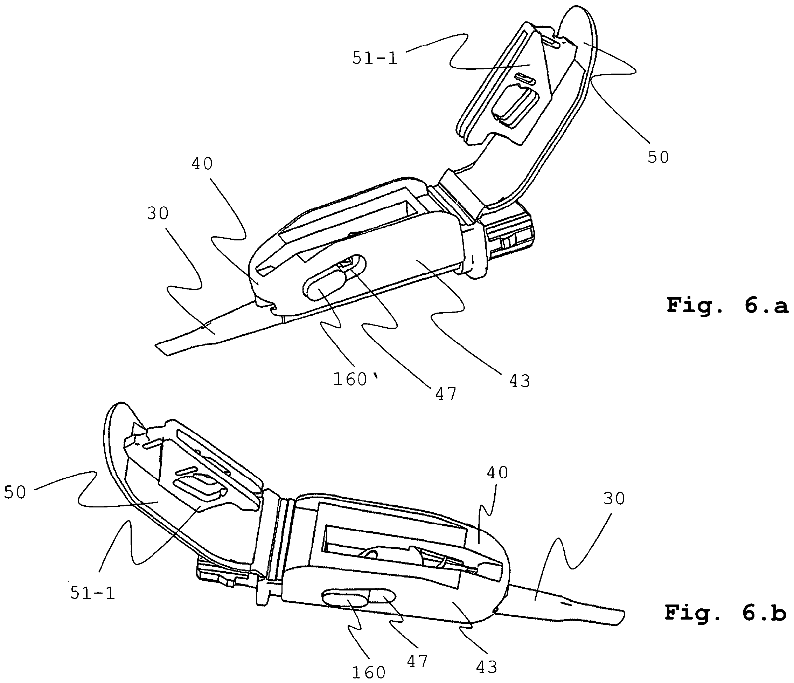

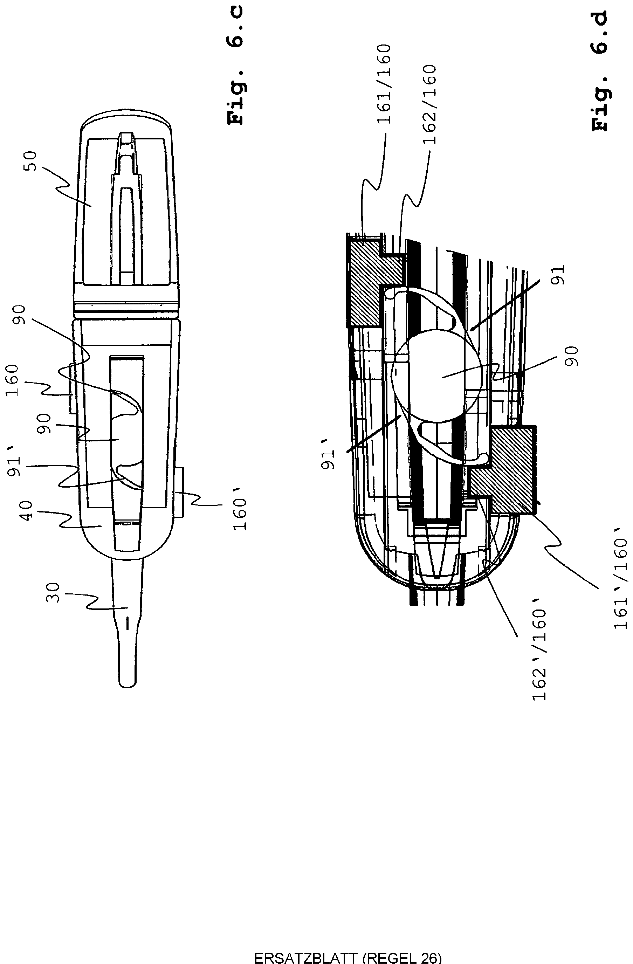

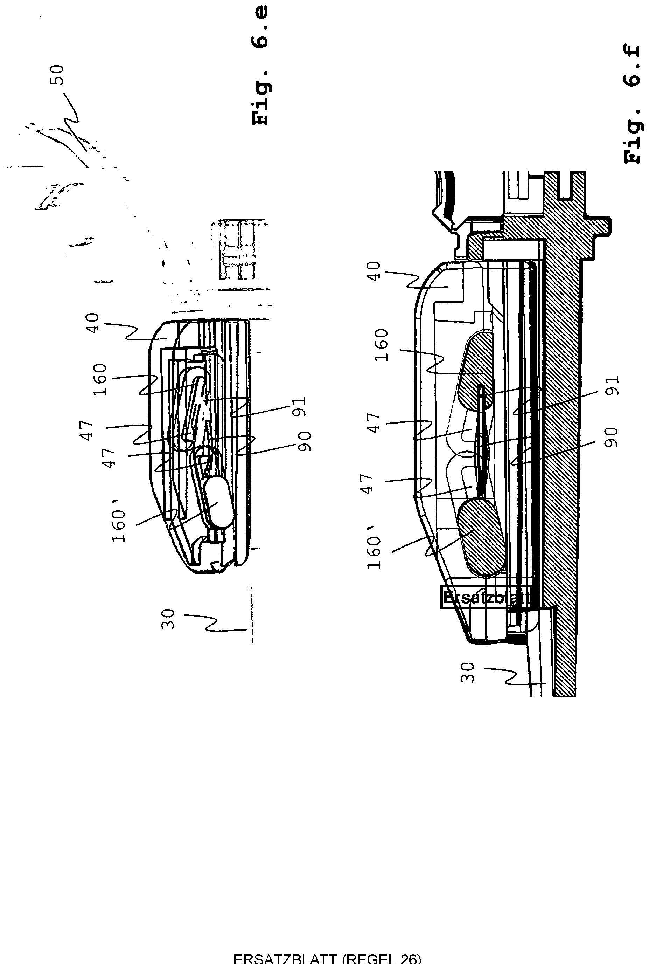

Die Figuren 6.a bis 6.f zeigen eine zweite Ausführungsform eines Magazins 40 mit zwei seitlich an den Magazinwänden 43 angebrachten Haptikschiebern 160 und 160'. Der Schieber 160 ist der hinteren Haptik 91 und der Schieber 160' ist der vorderen Haptik 91' zugeordnet. Das hier zugrundeliegende Funktionsprinzip entspricht im Wesentlichen dem der vorab beschriebenen ersten Ausführungsform. Hier erstrecken sich die beiden Haptikschieber 160 und 160' jeweils durch eine Öffnung 47 in der Seitenwand 43 des Magazins 40 (siehe dazu insbesondere die Figur 6.d).

Die beiden Haptikschieber 160 und 160' weisen dazu jeweils einen äußeren Abschnitt 161 bzw. 161' und einen inneren Abschnitt 162 bzw. 162' auf. Der jeweilige äußere Abschnitt 161 bzw. 161' dient dabei als Anlagefläche für die Finger des Anwenders zum Verschieben des Haptikschiebers 160 bzw. 160'. Der jeweilige innere Abschnitt 162 und 162' erstreckt sich durch die Öffnung 47 in der Magazinwand 43. Er stellt den Mitnehmer für die jeweilige Haptikspitze bereit.

Die beiden Öffnungen 47 sind hier jeweils zur Mitte des Magazins 40 hin ansteigend. Sie bilden eine Rampe für die beiden Haptikschieber 160 und 160'. Dadurch können die zwei Haptiken 91 und 91' gezielt auf bzw. an die Oberseite der Linse 90 geführt werden. Die Figuren 6.a bis 6.e zeigen die zwei Haptikschieber 160 und 160' jeweils in ihrem initialen Zustand mit noch nicht verschobenen Haptiken 91 und 91'.

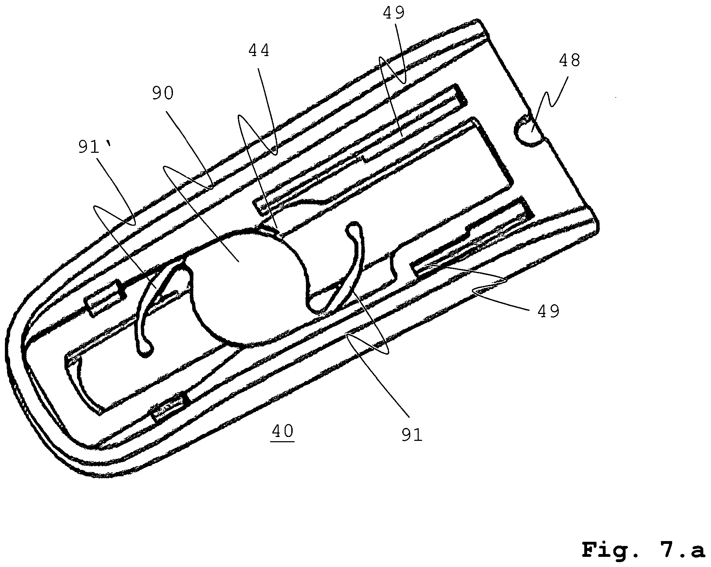

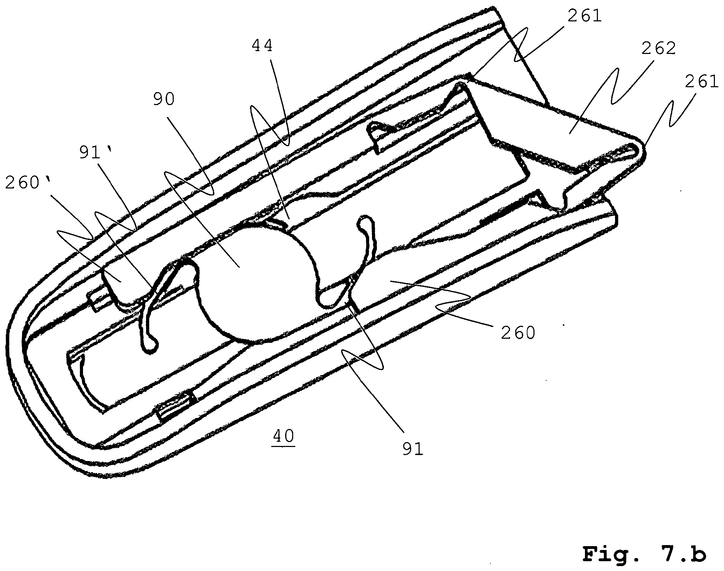

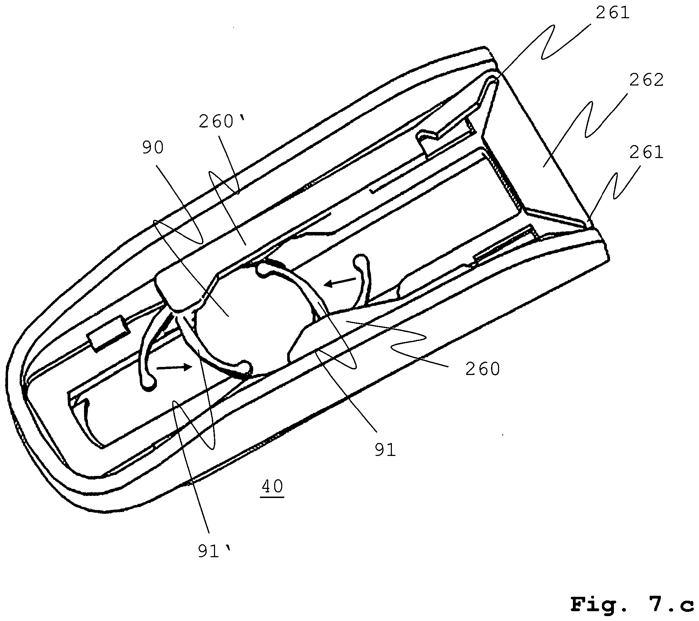

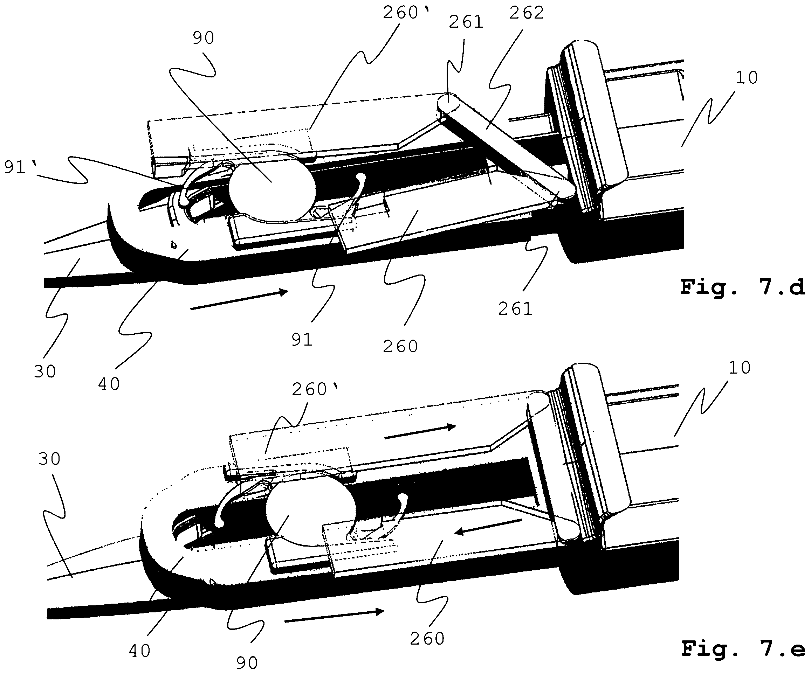

Die Figuren 7.a bis 7.e zeigen eine dritte Ausführungsform eines Magazins 40 mit zwei Haptikschiebern 260 und 260'. Die Bewegung der beiden Haptikschieber 260 und 260' ist in dieser Ausgestaltung gekoppelt. Zunächst ist in Figur 7.a das Magazin 40 noch ohne die beiden Haptikschieber 260 und 260' gezeigt. Die Linse 90 liegt in ihrem Aufnahmebereich 44 in dem Magazin 40. An der hinteren Seite ist im Magazin 40 eine Aussparung 48 eingebracht. Die Aussparung 48 stellt den Drehpunkt für ein Verbindungselement 262 bereit.

Das Verbindungselement 262 und die beiden Haptikschieber 260 und 260' sind in der Figur 7.b in ihrem Ausgangszustand dargestellt. Die beiden Endabschnitte der Haptikschieber 260 und 260' sind axial unterschiedlich positioniert. Das Verbindungselement 262 ist jeweils über ein Drehgelenk 261 mit den beiden Haptikschiebern 260 und 260' verbunden. Das Verbindungselement 262 stellt somit über ein Doppelgelenk eine Verbindung zwischen den beiden Haptikschiebern 260 und 260' bereit. Das Verbindungselement 262 und die beiden Haptikschieber 260 und 260' können zum Beispiel einteilig ausgebildet sein. Die beiden Gelenke 261 können in einer solchen Ausgestaltung zum Beispiel jeweils durch ein Folienscharnier bereitgestellt sein. An einer Unterseite des Verbindungselements 262 kann insbesondere ein Zapfen angeordnet sein (nicht in den Figuren gezeigt), der zur Bereitstellung des Drehpunkts in die Aussparung 48 im Magazin 40 eingreift.

Die zwei Haptikschieber 260 und 260' können in dem Magazin 40 zum Beispiel jeweils in oder auf einer Art Schiene 49, insbesondere axial, beweglich befestigt sein. Die Schiene 49 wird hier jeweils durch eine Nut bereitgestellt (siehe dazu Figur 7.a). Die Schienen 49 ermöglichen eine geführte Bewegung der beiden Haptikschieber 260 und 260'.

Die beiden Haptikschieber 260 und 260' greifen vorliegend an den Haptikansätzen an. Dadurch muss zwar eine größere Kraft zum Verschieben der Haptiken 91 und 91' aufgebracht werden. Dadurch wird aber auch der Weg, den die beiden Schieber 260 und 260' zurücklegen müssen, verkürzt.

Das Verbindungselement 262 ermöglicht über das Doppelgelenk 261 eine gekoppelte Bewegung der beiden Haptikschieber 260 und 260'. Figur 7.c zeigt den Endzustand. Die zwei Haptiken 91 und 91' sind jeweils in ihrem Anfangszustand sowie in ihrem Endzustand, in welchem sie an der Linse 90 anliegen, dargestellt. Die Überführung in den Endzustand bzw. die Bewegung der Haptikschieber 260 und 260' erfolgt hier durch das Aufschieben des Magazins 40 auf die Kanüle 30. Das ist noch einmal in den Figuren 7.d und 7.e dargestellt.

Die Figuren 7.d und 7.e illustrieren das zugrundliegende Prinzip dieser Ausführungsform. Dazu ist hier zusätzlich die Kanüle 30 des Injektors 100 dargestellt. Zum Laden des Injektors 100 wird das mit der Linse 90 bestückte Magazin 40 auf die Kanüle 30 aufgeschoben. Die Schubrichtung ist mit dem Pfeil angedeutet (Figur 7.d). Der hinten liegende Teil des Verbindungselements 262 kommt zur Anlage an einem Endabschnitt der Kanüle 30. Bei einem weiteren Aufschieben des Magazins 40 auf die Kanüle 40 bewirkt das Verbindungselement 262 durch die doppelgelenkige Verbindung 261 eine Bewegung der beiden Haptikschieber 260 und 260' relativ zu den Seitenwänden 43 des Magazins. Zum einen wird der Schieber 260 in Richtung der Vorderseite der Kanüle 30 verschoben und schiebt dabei die hintere Haptik 91 an bzw. auf die Linse 90. Zum anderen wird zeitgleich der Schieber 260' in Richtung der Rückseite der Kanüle 30 verschoben und schiebt dabei die vordere Haptik 91' an bzw. auf die Linse 90.

In Figur 7.e ist das Magazin 40 vollständig auf die Kanüle 30 aufgeschoben. Das Verbindungselement 262 liegt in diesem Zustand vollständig an dem Endabschnitt der Kanüle 30 an. Die beiden Haptikschieber 260 und 260' sind hier in ihrer finalen Position. Die beiden Haptiken 91 und 91' liegen an der Linse 90 an. Die verschobene finale Position der beiden Haptiken ist in der Figur 7.e jedoch nicht dargestellt.

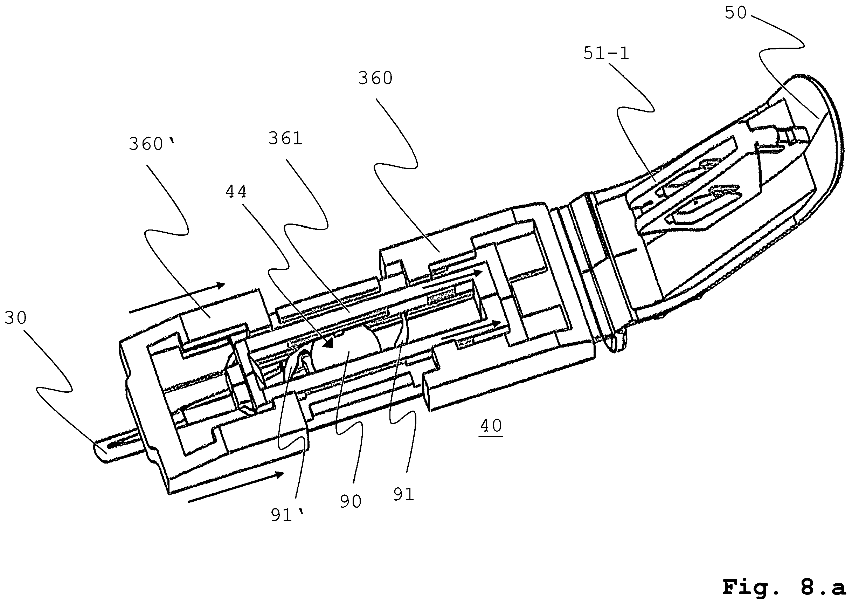

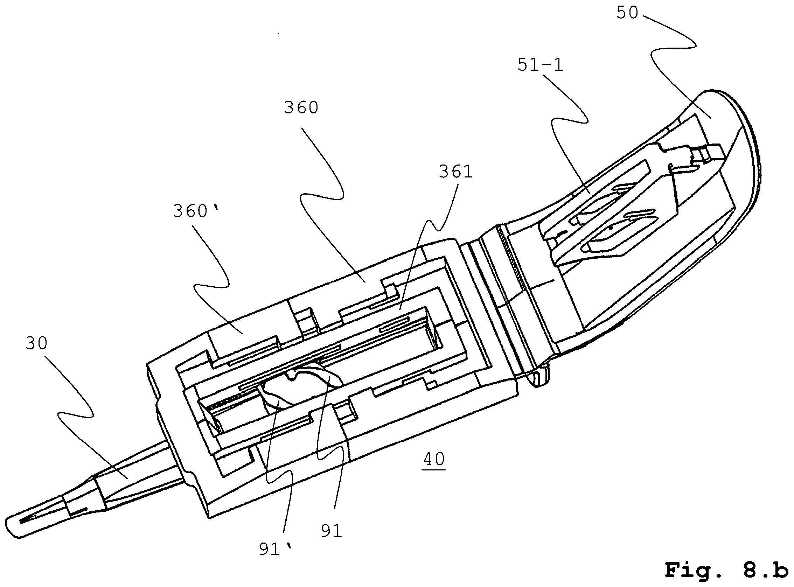

Abschließend zeigen die Figuren 8.a und 8.b eine vierte Ausführungsform eines Magazins 40 mit zwei Haptikschiebern 360 und 360'. Das Magazin 40 ist bereits auf die Kanüle 30 des Injektors 100 aufgeschoben. Figur 8.a zeigt die beiden Haptikschieber 360 und 360' in ihrem Ausgangszustand, Figur 8.b in ihrem Endzustand.

Die beiden Haptikschieber 360 und 360' sind beweglich an dem Magazin 40 montiert. Sie sind auf den Seitenwänden 43 des Magazins 40 aufgesetzt. Der hintere Haptikschieber 360 wird in Bezug auf die Kanüle 30 selbst nicht bewegt. Der Aufnahmebereich 44 für die Linse 90 ist in einem Schlitten 361 angeordnet. Der Schlitten 361 kann axial gegenüber der Kanüle 30 verschoben werden Zunächst wird der vordere Haptikschieber 360' in Richtung der Rückseite des Injektors 100 verschoben. Er kommt dabei zur Anlage an der vorderen Haptik 91' und verschiebt diese in Richtung der Linse 90. Zudem kommt dann der vordere Haptikschieber 360' zur Anlage an dem Schlitten 361. Durch das weitere Schieben des vorderen Haptikschiebers 360' wird somit auch der Schlitten 361, in welchem die Linse 90 positioniert ist, in Richtung des hinteren Haptikschiebers 360 verschoben. Die hintere Haptik 91 kommt dadurch zur Anlage an dem hinteren Haptikschieber 360. Die hintere Haptik 91 wird dadurch nach vorne in Richtung der Linse 90 verschoben.

Figur 8.b zeigt den Endzustand des Magazins 40, in welchem der vordere Haptikschieber 360' als auch der Schlitten 361 an dem hinteren Haptikschieber 360 anliegen. Die beiden Haptiken 91 und 91' liegen an der Linse 90 an bzw. auf der Linse 90 auf.

Es ist dem Fachmann ersichtlich, dass die beschriebenen Ausführungsformen beispielhaft zu verstehen sind. Die Erfindung ist nicht auf diese beschränkt, sondern kann in vielfältiger Weise variiert werden, ohne den Geist der Erfindung zu verlassen.

Merkmale einzelner Ausführungsformen und die im allgemeinen Teil der Beschreibung genannten Merkmale können jeweils untereinander als auch miteinander kombiniert werden.

### Bezugszeichenliste:

- 10: Injektorkörper oder Injektorgehäuse oder Gehäuse oder Handstück
- 11: Griff am Injektorkörper

- 20: Schieber oder Kolben oder Linsenschieber
- 21: Griff oder Schiebergriff

- 30: Kanüle oder Einführrohr in das Auge oder Ausführkörper für die Linse
- 31: Transportkanal oder Vorschubkanal
- 33: Vorsprung an der Kanüle oder Rastnase

- 40: Magazin oder Behälter zum Lagern der Linse
- 43: Seitenwand des Magazins
- 44: Aufnahmebereich für eine Linse
- 44a: Köpfe oder Erhebungen in dem Aufnahmebereich
- 45: Rampe oder Führungsbereich für eine Linsenhaptik
- 46: Schiene oder Führungsschiene für den Haptikschieber
- 47: Öffnung in der Seitenwand des Magazins
- 48: Aussparung in dem Magazin
- 49: Schiene oder Nut in dem Magazin

- 50: Faltklappe oder Faltplattenträger oder Klappe
- 51: Faltkörper oder Faltplatte oder Faltrippe
- 51-1: Sockel des Faltkörpers
- 52-2: Faltelement des Faltkörpers
- 52: Rückhaltekante an der Faltplatte
- 52d: Unterseite der Rückhaltekante

- 60: Haptikschieber (für die hintere Haptik)
- 60': Haptikschieber (für die vordere Haptik)
- 61: Vorsprung am Haptikschieber
- 62: Aufnahmebereich und Mitnehmer für die Haptikspitze
- 63: Äußerer Abschnitt des Haptikschiebers
- 64: Innerer Abschnitt des Haptikschiebers
- 65: Kopfabschnitt des Haptikschiebers
- 66: Vorsprung oder Rastvorsprung

- 90: Linse oder Intraokularlinse oder Linsenoptik
- 91: (Hintere) Haptik der Linse
- 91': (Vordere) Haptik der Linse

- 100: Injektor oder Injektorsystem oder Applikator
- 100a: Vorderseite des Injektors
- 100b: Rückseite des Injektors

- 160: Haptikschieber (für die hintere Haptik)
- 160': Haptikschieber (für die vordere Haptik)
- 161: Äußerer Bereich oder Abschnitt des (hinteren) Haptikschiebers
- 161': Äußerer Bereich oder Abschnitt des (vorderen) Haptikschiebers
- 162: Innerer Bereich oder Abschnitt des (hinteren) Haptikschiebers und Mitnehmer für die Haptikspitze
- 162': Innerer Bereich oder Abschnitt des (vorderen) Haptikschiebers und Mitnehmer für die Haptikspitze

- 260: Haptikschieber (für die hintere Haptik)
- 260': Haptikschieber (für die vordere Haptik)
- 261: Drehgelenk oder Scharnier
- 262: Verbindungselement (zwischen den Haptikschiebern)

- 360: Haptikschieber (für die hintere Haptik)
- 360': Haptikschieber (für die vordere Haptik)
- 361: Schlitten in dem Magazin

## Patentansprüche

1. Magazin (40) für ein Injektorsystem (100) zum Implantieren einer Linse (90) in ein Auge umfassend einen Aufnahmebereich (44) für wenigstens eine Linse (90) und zumindest einen bewegbaren Haptikschieber (60, 60', 160, 160', 260, 260', 360, 360') zum Verschieben einer Linsenhaptik (91, 91') in dem Magazin (4), insbesondere zum Schieben einer Linsenhaptik (91, 91') auf und/oder an eine Optik der Linse (90).

2. Magazin (40) nach vorstehendem Anspruch, **dadurch gekennzeichnet, dass** die Linse (90) in dem Aufnahmebereich (44) für die Linse (90) mittels des Haptikschiebers (60, 60', 260, 260') festsetzbar ist.

3. Magazin (40) nach einem der vorstehenden Ansprüche, **dadurch gekennzeichnet, dass** der Haptikschieber (60, 60', 260, 260') derart ausgeführt ist, dass die Linse (90) sowohl in einem initialen als auch in einem verschobenen Zustand des Haptikschiebers (60, 60') in dem Aufnahmebereich (44) festgesetzt ist.

4. Magazin (40) nach einem der vorstehenden Ansprüche, **dadurch gekennzeichnet, dass** der Haptikschieber (60, 60', 160, 160') einen Mitnehmer (62, 162, 162') für eine Haptikspitze der Linsenhaptik (91, 91') bereitstellt, welcher vorzugsweise durch eine Aussparung in einem inneren Abschnitt (64) des Haptikschiebers (60, 60') bereitgestellt ist.

5. Magazin (40) nach einem der vorstehenden Ansprüche, **dadurch gekennzeichnet, dass** auf einem Kopfabschnitt (65) oder einem äußeren Abschnitt (161, 161') des Haptikschiebers (60, 60', 160, 160') eine Erhebung (61) zum Verschieben des Haptikschiebers (60, 60', 160, 160') ausgebildet ist.

6. Magazin (40) nach einem der vorstehenden Ansprüche, **dadurch gekennzeichnet,**
**dass** der Haptikschieber (60, 60', 160, 160', 260, 260', 360, 360') beweglich an dem Magazin (40), vorzugsweise mittels einer Rastverbindung, montiert ist und/oder
**dass** der Haptikschieber (60, 60', 260, 260', 360, 360') auf einer Seitenwand (43) und/oder auf dem Boden des Magazins (40) im Wesentlichen axial verschiebbar angeordnet, vorzugsweise einrastend aufgesteckt, ist,

7. Magazin (40) nach einem der vorstehenden Ansprüche, **dadurch gekennzeichnet, dass** der Haptikschieber (160, 160') an einer Seitenwand (43) des Magazins (40) montiert ist und sich durch eine Öffnung (47) in der Seitenwand (43) des Magazins (40) in das Magazin (40) hinein erstreckt.

8. Magazin (40) nach einem der vorstehenden Ansprüche, **dadurch gekennzeichnet,**
**dass** das Magazin (40) einen hinteren Haptikschieber (60, 160, 260, 360), welcher einer hinteren Haptik (91) zugeordnet ist, und einen vorderen Haptikschieber (60', 160', 260', 360'), welcher einer vorderen Haptik (91') zugeordnet ist, aufweist und/oder
**dass** der hintere Haptikschieber (60, 160, 260, 360) und der vordere Haptikschieber (60', 160', 260', 360') in entgegengesetzte Richtungen verschiebbar sind.

9. Magazin (40) nach einem der vorstehenden Ansprüche, **dadurch gekennzeichnet, dass** der hintere Haptikschieber (60, 160, 260, 360) zum Schieben der hinteren Haptik (91), vorzugsweise auf oder an die Optik der Linse (90), in Richtung einer Vorderseite (100a) des Injektors (100) verschiebbar ist und/oder der vordere Haptikschieber (60', 160', 260', 360') zum Schieben der vorderen Haptik (91'), vorzugsweise auf oder an die Optik der Linse (90), in Richtung einer Rückseite (100b) des Injektors (100) verschiebbar ist.

10. Magazin (40) nach einem der vorstehenden Ansprüche, **dadurch gekennzeichnet, dass** der vordere Haptikschieber (260', 360') und der hintere Haptikschieber (260, 360) derart gekoppelt sind, dass durch eine einzige Betätigung sowohl die vordere Haptik (91') als auch die hintere Haptik (91) auf und/oder an die Optik der Linse (90) schiebbar sind.

11. Magazin (40) nach einem der vorstehenden Ansprüche, **gekennzeichnet**
**durch** einen beweglich, vorzugsweise drehbar, gelagerten Verbindungsabschnitt (262), der jeweils über ein Drehgelenk (261) mit dem vorderen Haptikschieber (260') und dem hinteren Haptikschieber (260) verbunden ist und/oder
**durch** einen axial verschiebbaren Schlitten (361), in welchem der Aufnahmebereich (44) für die Linse (90) angeordnet ist, wobei der Schlitten (361) durch eine axiale Verschiebung des vorderen Haptikschieber (360') in Richtung des hinteren Haptikschiebers (360) verschiebbar ist.

12. Magazin (40) nach einem der vorstehenden Ansprüche, **dadurch gekennzeichnet,**
**dass** an einem Abschnitt, vorzugsweise an einer Innenseite, der Seitenwände (43) des Magazins (40) jeweils eine Rampe (45) bereitgestellt ist und/oder
**dass** jeweils eine von der Vorderseite des Magazins (40) zunächst ansteigende und dann in Richtung der Rückseite des Magazins (40) wieder abfallende Rampe (45) bereitgestellt ist, insbesondere
wobei der Aufnahmebereich (44) für die wenigstens eine Linse (90) jeweils durch eine, vorzugsweise in etwa mittig angeordnete, Aussparung in der Rampe (45) bereitgestellt ist.

13. Magazin (40) nach einem der vorstehenden Ansprüche, **dadurch gekennzeichnet,**
**dass** eine Haptikspitze der vorderen Linsenhaptik (91') und eine Haptikspitze der hinteren Linsenhaptik (91) in einem initialen Zustand jeweils neben dem Aufnahmebereich (44) für die Linse (90) auf der Rampe (45) aufliegen können und/oder
**dass** in dem Aufnahmebereich (44) für die Linse (90) jeweils zumindest zwei Köpfe (44a) angeordnet sind, auf denen jeweils die Linsenoptik, vorzugsweise ein Randbereich der Linsenoptik (90), aufliegen kann und/oder
**dass** eine Linse (90) in dem Aufnahmebereich (44) des Magazins (40) im Wesentlichen spannungsfrei oder vorgefaltet gelagert ist.

14. Injektorsystem (100) zum Implantieren einer Linse (90) in ein Auge umfassend
- einen Injektorkörper (10) mit einer Vorderseite (10a) und einer Rückseite (10b),
- eine an der Vorderseite des Injektorkörpers (10) angeordnete Kanüle (30), die einen Transportkanal (31) für eine zu implantierende Linse (90) bereitstellt, wobei dem Transportkanal (31) eine Linse (90) über eine vorzugsweise seitliche Eingangsöffnung (32) zuführbar ist,
- ein Magazin (40) nach einem der vorstehenden Ansprüche 1 bis 13 mit einem Aufnahmebereich (44) für wenigstens eine Linse (90), wobei das Magazin (40) so angeordnet ist, dass dem Transportkanal (31) eine Linse (90) über die vorzugsweise seitliche Eingangsöffnung (32) zuführbar ist,
- einen in das Magazin (40) und die Eingangsöffnung (32) einfügbaren Faltkörper (51) zum Einschieben der Linse (90) in den Transportkanal (32) derart, dass die Linse (90) zumindest abschnittsweise um den Faltkörper (51) faltbar ist, und
- einen Schieber (20), welcher im Injektorkörper (10), vorzugsweise axial, verschiebbar angeordnet ist und über die Vorderseite des Injektorkörpers (10) so in den Transportkanal (31) einschiebbar ist, dass die Linse (90) aus dem Transportkanal (31) ausgeschoben werden kann.

15. Injektorsystem (100) nach vorstehendem Anspruch, **dadurch gekennzeichnet, dass**
das Magazin (40) zum Laden des Injektorsystems (100) mit einer Linse (90) mit der Kanüle (30) verbindbar ist, vorzugsweise auf die Kanüle (30) des Injektors (100) so aufschiebbar oder aufsteckbar ist, dass die Kanüle (30) zumindest abschnittsweise im Inneren (41) des Magazins (40) oder am Magazin (40) angeordnet ist.

## Claims

1. . A cartridge (40) for an injector system (100) for implanting a lens (90) into an eye, comprising a receiving region (44) for at least one lens (90) and at least one movable haptic slider (60, 60', 160, 160', 260, 260', 360, 360') for displacing a lens haptic (91, 91') within the cartridge (4), in particular for sliding a lens haptic (91, 91') onto and/or or to an optical portion of the lens (90).

2. . The cartridge (40) as claimed in the preceding claim, wherein the lens (90) can be captured within the receiving region (44) for the lens (90) by means of the haptic slider (60, 60', 260, 260').

3. . The cartridge (40) as claimed in any of the preceding claims, wherein the haptic slider (60, 60', 260, 260') is adapted such that the lens (90) is captured within the receiving region (44) in both, an initial state and a displaced state of the haptic slider (60, 60').

4. . The cartridge (40) as claimed in any of the preceding claims, wherein the haptic slider (60, 60', 160, 160') provides a driver (62, 162, 162') for a haptic tip of the lens haptic (91, 91'), which is preferably provided by a recess in an inner portion (64) of the haptic slider (60, 60').

5. . The cartridge (40) as claimed in any of the preceding claims, comprising a projection for moving the haptic slider, which projection is formed on a head portion (65) or on an outer portion (161, 161') of the haptic slider (60, 60', 160, 160').

6. . The cartridge (40) as claimed in any of the preceding claims, wherein the haptic slider (60, 60', 160, 160', 260, 260', 260, 360, 360') is mounted to the cartridge (40) so as to be movable, preferably by a snap-in connection and/or wherein the haptic slider (60, 60', 260, 260', 360, 360') is arranged on, preferably latched to a sidewall (43) or a bottom of the cartridge (40) so as to be displaceable substantially axially.

7. . The cartridge (40) as claimed in any of the preceding claims, wherein the haptic slider (160, 160') is mounted to a sidewall (43) of the cartridge (40) and extends into the cartridge (40) through an opening (47) in the sidewall (43) of the cartridge (40).

8. . The cartridge (40) as claimed in any of the preceding claims, wherein the cartridge has a rear haptic slider (60, 160, 260, 360) associated with a rear haptic (90), and a front haptic slider (60', 160', 260', 360') associated with a front haptic (91'),and/or wherein the rear haptic slider and the front haptic slider are displaceable in opposite directions.

9. . The cartridge (40) as claimed in any of the preceding claims, wherein the rear haptic slider (60, 160, 260, 360) for sliding the rear haptic (91), preferably onto or to the optical portion of the lens (90), is displaceable towards a front end (100a) of the injector (100), or wherein the front haptic slider for sliding the front haptic (60', 160', 260', 360') preferably onto or to the optical portion of the lens, is displaceable towards a rear end (100b) of the injector (100) .

10. . The cartridge (40) as claimed in any of the preceding claims, wherein the front haptic slider (260', 360')and the rear haptic slider (260, 360) are coupled such that both the front haptic (91') and the rear haptic (91) can be slid onto or to the optical portion of the lens (90) by a single actuation.

11. . The cartridge (40) as claimed in any of the preceding claims, comprising a link member (262) mounted for being moved, preferably rotated, which is connected to each of the front haptic slider (260') and the rear haptic slider (260) through a respective rotary joint (261),
and/or comprising an axially displaceable carriage (361) wherein the receiving region (44) for the lens (90) is provided, wherein said carriage (361) is displaceable towards the rear haptic slider (360) by an axial displacement of the front haptic slider (360').

12. . The cartridge (40) as claimed in any of the preceding claims, wherein a respective ramp is provided on a portion of the cartridge (40), preferably on an inner surface of the sidewalls (43) thereof,
and/or wherein a respective ramp (45) is provided having an initial upslope from the front end of the cartridge (40) and then a downslope (43) towards the rear end of the cartridge (40),in particular wherein the receiving region (44) for the at least one lens (90) is provided by a respective recess in the ramp (45) which is preferably disposed approximately centrally.

13. . The cartridge (40= as claimed in any of the preceding claims, wherein in an initial state each of a haptic tip of the front lens haptic (91') and a haptic tip of the rear lens haptic (91) can be supported on the ramp next to the receiving region (44) for the,
and/or wherein at least two heads (44a) are provided in each receiving region (44) for the lens (90), which are adapted to support the optical portion of the lens (90), preferably a peripheral area of the optical portion of the lens (90), and/or wherein a lens (90) is stored within the receiving region(44) of the cartridge in a substantially stress-free state or in a pre-folded state.

14. . An injector system (100) for implanting a lens (90) into an eye, comprising:
an injector body (10) having a front end (100a) and a rear end (100b);
a cannula (30) arranged at the front end of the injector body (10), which provides a transport channel (31) for a lens to be implanted (90), wherein a lens can be fed into the transport channel via a preferably lateral inlet opening (32);
a cartridge (110), comprising:
a receiving region (44) for at least one lens (90) and at least one movable haptic slider(31) for displacing a lens (90) haptic within the cartridge, in particular for sliding a lens haptic onto or to an optical portion of the lens, wherein the cartridge is arranged such that a lens can be fed into the transport channel (32) via the preferably lateral inlet opening (32);
a folding body which is insertable into the cartridge and into the inlet opening, for pushing the lens (90) into the transport channel (32) in such a manner that the lens is foldable around the folding body, at least portions thereof; and
a slider (20) which is arranged within the injector body (10) so as to be displaceable, preferably axially, and which can be pushed through the front end of the injector body (10) and into the transport channel (31) in such a way that the lens (90) can be ejected from the transport channel (31).

15. . The injector system (100) as claimed in the preceding claim, wherein for loading the injector system (100) with a lens (90), the cartridge (40) can be connected with the cannula (30), preferably slidingly fitted or plugged onto the cannula (30) of the injector system (100) in a manner so that the cannula (30) is arranged in the interior (41) of the cartridge (40), at least a portion thereof, or on the cartridge (40).

## Revendications

1. Chargeur (40) pour un système d'injection (100) destiné à implanter une lentille (90) dans un œil, comprenant une zone de réception (44) pour au moins une lentille (90) et au moins un coulisseau de prise mobile (60, 60', 160, 160', 260, 260', 360, 360') pour déplacer une prise de lentille (91, 91') dans le chargeur (40), en particulier pour faire coulisser une prise de lentille (91, 91') sur et/ou à une optique lenticulaire (90).

2. Chargeur (40) selon la revendication précédente, **caractérisé en ce que** la lentille (90) peut être fixée dans la zone de réception (44) pour la lentille (90) au moyen du coulisseau de prise (60, 60', 260, 260').

3. Chargeur (40) selon l'une des revendications précédentes, **caractérisé en ce que** le coulisseau de prise (60, 60', 260, 260') est conçu de manière à ce que la lentille (90) se fixe dans la zone de réception (44) dans un état initial comme dans un état déplacé du coulisseau de prise (60, 60').

4. Chargeur (40) selon l'une des revendications précédentes, **caractérisé en ce que** le coulisseau de prise (60, 60', 160, 160') fournit un organe d'entraînement (62, 162, 162') pour une pointe de prise de la prise de lentille (91, 91'), lequel est préférablement fourni par une encoche dans une section intérieure (64) du coulisseau de prise (60, 60').

5. Chargeur (40) selon l'une des revendications précédentes, **caractérisé en ce qu'**une élévation (61) pour le déplacement du coulisseau de prise (60, 60', 160, 160') est formée sur une section supérieure (65) ou une section extérieure (161, 161') du coulisseau de prise (60, 60', 160, 160').

6. Chargeur (40) selon l'une des revendications précédentes, **caractérisé**
**en ce que** le coulisseau de prise (60, 60', 160, 160', 260, 260', 360, 360') est monté de façon à être mobile sur le chargeur (40), préférablement au moyen d'une liaison par encliquetage, et/ou
**en ce que** le coulisseau de prise (60, 60', 260, 260', 360, 360') est disposé de façon à pouvoir être déplacé essentiellement axialement sur une paroi latérale (43) et/ou sur le fond du chargeur (40), préférablement est embroché par encliquetage.

7. Chargeur (40) selon l'une des revendications précédentes, **caractérisé en ce que** le coulisseau de prise (160, 160') est monté sur une paroi latérale (43) du chargeur (40) et s'étend dans le chargeur (40) par un orifice (47) situé dans la paroi latérale (43) du chargeur (40).

8. Chargeur (40) selon l'une des revendications précédentes, **caractérisé**
**en ce que** le chargeur (40) présente un coulisseau de prise arrière (60, 160, 260, 360) qui est affecté à une prise arrière (91), et un coulisseau de prise avant (60', 160', 260', 360') qui est affecté à une prise avant (91'), et/ou en ce que le coulisseau de prise arrière (60, 160, 260, 360) et le coulisseau de prise avant (60', 160', 260', 360') sont coulissants dans des directions opposées.

9. Chargeur (40) selon l'une des revendications précédentes, **caractérisé en ce que** le coulisseau de prise arrière (60, 160, 260, 360) pour le déplacement de la prise arrière (91) est déplaçable, préférablement sur ou à l'optique lenticulaire (90), en direction d'une partie avant (100a) de l'injecteur (100), et/ou le coulisseau de prise avant (60', 160', 260', 360') pour le déplacement de la prise avant (91') est déplaçable, préférablement sur ou à l'optique lenticulaire (90), en direction d'une partie arrière (100b) de l'injecteur (100).

10. Chargeur (40) selon l'une des revendications précédentes, **caractérisé en ce que** le coulisseau de prise avant (260', 360') et le coulisseau de prise arrière (260, 360) sont couplés de telle manière à ce que, par un seul actionnement, la prise avant (91') et la prise arrière (91) puissent être déplacées sur et/ou à l'optique lenticulaire (90).

11. Chargeur (40) selon l'une des revendications précédentes, **caractérisé**
**par** une section de liaison (262) mobile, préférablement pivotante, qui est reliée respectivement au coulisseau de prise avant (260') et au coulisseau de prise arrière (260) par une articulation rotative (261) et/ou
par une glissière (361) coulissante axialement, dans laquelle est disposée la zone de réception (44) pour la lentille (90), la glissière (361) étant coulissante par un déplacement axial du coulisseau de prise avant (360') en direction du coulisseau de prise arrière (360).

12. Chargeur (40) selon l'une des revendications précédentes, **caractérisé**
**en ce qu'**une rampe (45) est fournie sur une section, préférablement sur une partie intérieure des parois latérales (43) du chargeur (40) et/ou
**en ce qu'**une rampe (45) montant d'abord de la partie avant du chargeur (40), puis redescendant en direction de la partie arrière du chargeur (40), est fournie, en particulier la zone de réception (44) étant fournie pour au moins une lentille (90) respectivement par une encoche dans la rampe (45), placée préférablement à peu près au centre.

13. Chargeur (40) selon l'une des revendications précédentes, **caractérisé**
**en ce qu'**une pointe de prise de la prise de lentille avant (91') et une pointe de prise de la prise de lentille arrière (91) puissent reposer sur la rampe (45), respectivement dans un état initial à côté de la zone de réception (44) pour la lentille (90)
et/ou
**en ce que**, dans la zone de réception (44) pour la lentille (90), sont disposées respectivement au moins deux têtes (44a) sur lesquelles peut respectivement reposer l'optique lenticulaire, préférablement une zone marginale de l'optique lenticulaire (90), et/ou
**en ce qu'**une lentille (90) est essentiellement montée sans tension ou pré-pliée dans la zone de réception (44) du chargeur (40) .

14. Système d'injection (100) destiné à implanter une lentille (90) dans un œil, comprenant
- un corps d'injecteur (10) avec une partie avant (10a) et une partie arrière (10b),
- une canule (30) disposée sur la partie avant du corps d'injecteur (10), laquelle met à disposition un canal de transport (31) pour une lentille (90) à implanter, une lentille (90) pouvant être amenée au canal de transport (31) par un orifice d'entrée (32) préférablement latéral,
- un chargeur (40) selon l'une des revendications précédentes 1 à 13, avec une zone de réception (44) pour au moins une lentille (90), le chargeur (40) étant disposé de telle manière à ce qu'une lentille (90) puisse être amenée au canal de transport (31) par l'orifice d'entrée (32) préférablement latéral,
- un corps de pliage (51) pouvant être inséré dans le chargeur (40) et l'orifice d'entrée (32), pour l'insertion de la lentille (90) dans le canal de transport (32) de sorte que la lentille (90) puisse être pliée au moins en partie autour du corps de pliage (51),
- un coulisseau (20) qui est disposé de façon à pouvoir être déplacé, préférablement axialement, dans le corps d'injecteur (10) et qui peut être inséré dans le canal de transport (31) par la partie avant du corps d'injecteur (10) de façon à ce que la lentille (90) puisse être extraite du canal de transport (31).

15. Système d'injection (100) conformément à la revendication précédente, **caractérisé**
**en ce que** le chargeur (40) pour le chargement du système d'injection (100) avec une lentille (90) peut être relié à la canule (30), préférablement peut être monté ou emboîté sur la canule (30) de l'injecteur (100), de façon à ce que la canule (30) soit disposée au moins en partie à l'intérieur (41) du chargeur (40) ou au chargeur (40).
